# EUROPEAN PATENT SPECIFICATION

(11) **EP 2 529 620 B1**
(45) Date of publication and mention of the grant of the patent: **15.07.2020**
(21) Application number: 11737126.0
(22) Date of filing: 27.01.2011
(51) Int. Cl.: A01N 37/46, A01P 7/02, A01P 7/04, A61K 31/167, A61K 31/27, A61K 31/277, A61P 33/14, C07C 237/52, C07C 237/42

(54) **COMPOSITION FOR CONTROL OF ANIMAL PARASITES, AND METHOD FOR CONTROL OF ANIMAL PARASITES**
ZUSAMMENSETZUNG ZUR BEKÄMPFUNG VON TIERPARASITEN UND VERFAHREN ZUR BEKÄMPFUNG VON TIERPARASITEN
COMPOSITION ET PROCÉDÉ DE LUTTE CONTRE LES PARASITES DES ANIMAUX

(30) Priority: 29.01.2010 JP 2010019747
(43) Date of publication of application: 05.12.2012
(73) Proprietor: Mitsui Chemicals Agro, Inc., Tokyo 103-0027 (JP)
(72) Inventor: NOMURA, Michikazu, Mobara-shi Chiba 297-0017 (JP)
(74) Representative: Mewburn Ellis LLP
(86) International application number: PCT/JP2011/051673
(87) International publication number: WO 2011/093415

(56) References cited:
- EP-A1- 2 072 501
- EP-A1- 2 319 830
- WO-A1-2005/073165
- WO-A1-2010/018714
- WO-A1-2010/018857
- WO-A2-2009/080203
- JP-A- 2007 031 395

## Description

### Technical Field

The present invention relates to a composition for exterminating an animal parasite and a method for exterminating an animal parasite.

### Background Art

International Publication WO2005/21488, International Publication WO2005/73165, International Publication WO2006/137376, and International Publication WO2006/137395 disclose various compounds which are amide derivatives having the ability to control pests, and methods for using them. Further International Publication WO2009/080203 discloses amide derivatives which are animal parasiticides, and methods for using them.

In addition, conventional parasiticides for administration to an animal to exterminate animal parasites include formulations of imidacloprid, fipronil, etc.

WO2005/073165 discusses insecticides compounds similar to the compounds of the present invention. WO2005/073165 does not disclose methods of treatment or methods of killing parasites using the compounds similar to the present invention disclosed therein.

WO 2000/55120, WO 2000/7980 and US 2002-032238 each describe a compound similar to the compounds of the present invention. The compounds in these documents clearly do not fall within the scope of claims of the present invention. Additionally, none of the documents disclose parasiticidal activity of the compound.

### SUMMARY OF INVENTION

### Technical Problem

However, some parasites are impossible or difficult to exterminate with said animal parasiticides.

Thus, an objective of the invention is to provide a composition that has excellent activity for exterminating animal parasites, and a method for exterminating animal parasites.

### Solution to Problem

As the result of earnest research for solving the above problem, the present inventors found that an aromatic carboxamide derivative represented by Formula (1) of the present invention and a composition containing the present aromatic carboxamide derivative as an active ingredient have excellent activity for exterminating animal parasites, thereby completing the present invention.

That is, the present invention is as follows.
<1> A composition for exterminating animal parasites, which comprises, as an active ingredient, at least one amide derivative represented by the following Formula (1). 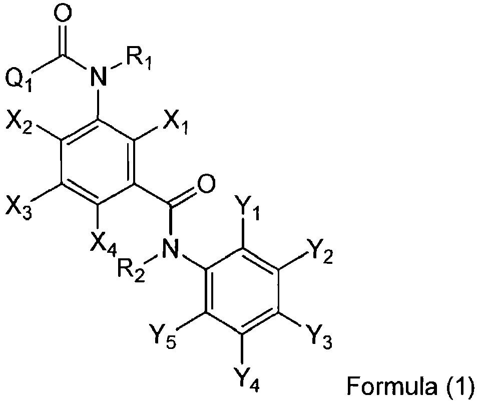 In Formula (1), Q₁ represents a phenyl group or a phenyl group substituted with a halogen atom; X₁ represents a fluorine atom, and X₂, X₃, and X₄ each represent a hydrogen atom; R₁ represents a hydrogen atom or a C₁-C₃ alkyl group; R₂ represents a hydrogen atom; Y₁ and Y₅ each independently represent a halogen atom or a C₁-C₃ haloalkyl group; Y₂ and Y₄ each represent a hydrogen atom; and Y₃ represents a heptafluoroisopropyl group.
<2> The composition according to <1>, wherein in Formula (1), Q₁ represents a phenyl group or a phenyl group substituted with a single fluorine atom; R₁ represents a hydrogen atom or a methyl group; and Y₁ and Y₅ each independently represent a bromine atom, an iodine atom or a trifluoromethyl group.
<3> The composition according to <2>, wherein the amide derivative represented by Formula (1) comprises 2-fluoro-3-(N-methylbenzamide)-N-(2-bromo-6-trifluoromethyl-4-(heptafluoropropan-2-yl)phenyl)benzamide, 2-fluoro-3-(4-fluoro-N-methylbenzamide)-N-(2-iodo-6-trifluoromethyl-4-(heptafluoropropan-2-yl)phenyl)benzamide, 2-fluoro-3-(3-fluoro-N-methylbenzamide)-N-(2-iodo-6-trifluoromethyl-4-(heptafluoropropan-2-yl)phenyl)benzamide, 2-fluoro-3-(4-fluorobenzamide)-N-(2-iodo-6-trifluoromethyl-4-(heptafluoropropan-2-yl)phenyl)benzamide, or 2-fluoro-3-(N-methylbenzamide)-N-(2,6-dibromo-4-(heptafluoropropan-2-yl)phenyl)benzamide.
<4> The composition according to <1>, wherein in Formula (1), Q₁ represents a phenyl group, a 2-fluorophenyl group, a 3-fluorophenyl group, a 4-fluorophenyl group, a 2-chlorophenyl group, a 3-chlorophenyl group, a 4-chlorophenyl group, a 2-bromophenyl group, a 3-bromophenyl group, a 4-bromophenyl group, a 2-iodophenyl group, a 3-iodophenyl group, or a 4-iodophenyl group.
<5> The composition according to <1>, wherein the amide derivative represented by Formula (1) comprises 3-benzamide-N-(2-bromo-6-chloro-4-(heptafluoropropan-2-yl)phenyl)-2-fluoro-benzamide, 3-benzamide-N-(2,6-diiodo-4-(heptafluoropropan-2-yl)phenyl)-2-fluorobenzamide, 3-benzamide-N-(2,6-dibromo-4-(heptafluoropropan-2-yl)phenyl)-2-fluorobenzamide, N-(2,6-dibromo-4-(heptafluoropropan-2-yl)phenyl)-2-fluoro-3-(N-methylbenzamide) benzamide, N-(2,6-dichloro-4-(heptafluoropropan-2-yl)phenyl)-2-fluoro-3-(N-methylbenzamide) benzamide, N-(2,6-diiodo-4-(heptafluoropropan-2-yl)phenyl)-2-fluoro-3-(N-methylbenzamide) benzamide, N-(2,6-diiodo-4-(heptafluoropropan-2-yl)phenyl)-2-fluoro-3-(2-fluorobenzamide) benzamide, N-(2,6-diiodo-4-(heptafluoropropan-2-yl)phenyl)-2-fluoro-3-(4-fluorobenzamide)benzamide, 3-(2,6-difluorobenzamide)-N-(2,6-diiodo-4-(heptafluoropropan-2-yl)phenyl)-2-fluoro benzamide, N-(2-bromo-6-iodo-4-(heptafluoropropan-2-yl)phenyl)-2-fluoro-3-(N-methylbenzamide) benzamide.
   N-(2,6-diiodo-4-(perfluoropropan-2-yl)phenyl)-2-fluoro-3-(3-fluorobenzamide)benzamide, N-(3-(2,6-diiodo-4-(heptafluoropropan-2-yl)phenylcarbamoyl)-2-fluorophenyl)-2-fluoro-N-methyl benzamide, N-(2,6-diiodo-4-(heptafluoropropan-2-yl)phenyl)-2-fluoro-3-(3-fluoro-N-methylbenzamide) benzamide, N-(2,6-diiodo-4-(heptafluoropropan-2-yl)phenyl)-2-fluoro-3-(4-fluoro-N-methylbenzamide) benzamide, N-(3-(2,6-diiodo-4-(heptafluoropropan-2-yl)phenylcarbamoyl)-2-fluorophenyl)-2,6-difluoro-N-methyl benzamide, N-(2,6-dibromo-4-(heptafluoropropan-2 -yl)phenyl)-2-fluoro-3 -(2-fluorobenzamide) benzamide, N-(2,6-dibromo-4-(heptafluoropropan-2-yl)phenyl)-2-fluoro-3-(3-fluorobenzamide) benzamide, N-(2,6-dibromo-4-(heptafluoropropan-2-yl)phenyl)-2-fluoro-3-(4-fluorobenzamide) benzamide, N-(2,6-dibromo-4-(heptafluoropropan-2-yl)phenyl)-3-(2,6-difluorobenzamide)-2-fluoro benzamide, N-(3-(2,6-dibromo-4-(heptafluoropropan-2-yl)phenylcarbamoyl)-2-fluorophenyl)-2-fluoro-N-methyl benzamide, N-(2,6-dibromo-4-(heptafluoropropan-2-yl)phenyl)-2-fluoro-3-(3-fluoro-N-methylbenzamide) benzamide, N-(2,6-dibromo-4-(heptafluoropropan-2-yl)phenyl)-2-fluoro-3-(4-fluoro-N-methylbenzamide) benzamide, N-(3-(2,6-dibromo-4-(heptafluoropropan-2-yl)phenylcarbamoyl)-2-fluorophenyl)-2,6-difluoro-N-methyl benzamide.
   3-benzamide-N-(2-bromo-4-(heptafluoropropan-2-yl)-6-(trifluoromethyl)phenyl)-2-fluorobenzamide, N-(2-bromo-4-(heptafluoropropan-2-yl)-6-(trifluoromethyl)phenyl)-2-fluoro-3-(N-methylbenzamide) benzamide, 3-benzamide-2-fluoro-N-(4-(heptafluoropropan-2-yl)-2,6-bis(trifluoromethyl)phenyl) benzamide, 3 -benzamide-2-fluoro-N-(2-iodo-4-(heptafluoropropan-2-yl)-6-(trifluoromethyl)phenyl) benzamide, 3-benzamide-N-(2-bromo-6-(pentafluoroethyl)-4-(heptafluoropropan-2-yl)phenyl)-2-fluorobenzamide, 2-fluoro-N-(2-iodo-4-(heptafluoropropan-2-yl)-6-(trifluoromethyl)phenyl)-3-(N-methylbenzamide) benzamide, 2-fluoro-N-(2-fluoro-3-(2-iodo-4-(heptafluoropropan-2-yl)-6-(trifluoromethyl)phenylcarbamoyl)phenyl)-N-methyl benzamide, 2-fluoro-3-(3-fluoro-N-methylbenzamide)-N-(2-iodo-4-(heptafluoropropan-2-yl)-6-(trifluoromethyl)phenyl) 2-fluoro-3-(4-fluoro-N-methylbenzamide)-N-(2-iodo-4-(heptafluoropropan-2-yl)-6-benzamide, (trifluoromethyl)phenyl)benzamide, 2,6-difluoro-N-(2-fluoro-3-(2-iodo-4-(heptafluoropropan-2-yl)-6-(trifluoromethyl)phenylcarbamoyl)phenyl)-N-methyl benzamide, 2-fluoro-3-(2-fluorobenzamide)-N-(2-iodo-4-(heptafluoropropan-2-yl)-6-(trifluoromethyl)phenyl) benzamide, 2-fluoro-3-(3-fluorobenzamide)-N-(2-iodo-4-(heptafluoropropan-2-yl)-6-(trifluoromethyl)phenyl) benzamide, N-(3-(2-bromo-4-(heptafluoropropan-2-yl)-6-(trifluoromethyl)phenylcarbamoyl)-2-fluorophenyl)-2-fluoro-N-methyl benzamide, N-(3-(2-bromo-4-(heptafluoropropan-2-yl)-6-(trifluoromethyl)phenylcarbamoyl)-2-fluorophenyl)-2,6-difluoro-N-methyl benzamide, N-(2-bromo-4-(heptafluoropropan-2-yl)-6-(trifluoromethyl)phenyl)-2-fluoro-3-(3-fluoro-N-methylbenzamide) benzamide, N-(2-bromo-4-(heptafluoropropan-2-yl)-6-(trifluoromethyl)phenyl)-2-fluoro-3-(4-fluoro-N-methylbenzamide) benzamide, N-(2-bromo-4-(heptafluoropropan-2-yl)-6-(trifluoromethyl)phenyl)-2-fluoro-3-(2-fluorobenzamide) benzamide, N-(2-bromo-4-(heptafluoropropan-2-yl)-6-(trifluoromethyl)phenyl)-2-fluoro-3-(3-fluorobenzamide) benzamide, N-(2-bromo-4-(heptafluoropropan-2-yl)-6-(trifluoromethyl)phenyl)-2-fluoro-3-(4-fluorobenzamide) benzamide.
   2-fluoro-3-(4-fluorobenzamide)-N-(2-iodo-4-(heptafluoropropan-2-yl)-6-(trifluoromethyl)phenyl)benzamide, 3-(2,6-difluorobenzamide)-2-fluoro-N-(2-iodo-4-(perfluoropropan-2-yl)-6-(trifluoromethyl)phenyl) benzamide, N-(2-bromo-4-(heptafluoropropan-2-yl)-6-(trifluoromethyl)phenyl)-3-(2,6-difluorobenzamide)-2-fluoro benzamide, 3-(2,4-dichlorobenzamide)-2-fluoro-N-(2-iodo-4-(heptafluoropropan-2-yl)-6-(trifluoromethyl)phenyl) benzamide, 3-(2-chloro-4-fluorobenzamide)-2-fluoro-N-(2-iodo-4-(heptafluoropropan-2-yl)-6-(trifluoromethyl)phenyl) benzamide, 3-(2-chlorobenzamide)-2-fluoro-N-(2-iodo-4-(heptafluoropropan-2-yl)-6-(trifluoromethyl)phenyl) benzamide, or N-(2-chloro-4-(heptafluoropropan-2-yl)-6-(trifluoromethyl)phenyl)-2-fluoro-3-(N-methylbenzamide) benzamide. Also disclosed herein is:
<6> A method for exterminating animal parasites comprising administering to an animal the composition for exterminating animal parasites disclosed in any one of clauses 1 to 5 above.
<7> The method for exterminating animal parasites disclosed in <6>, wherein the animal parasite is an ectoparasite.
<8> The method for exterminating animal parasites disclosed in <7>, wherein the ectoparasite is a Siphonaptera pest.
<9> The method for exterminating animal parasites disclosed in <7>, wherein the ectoparasite is an Acarina pest.

### Advantageous Effects of Invention

According to the present invention, a composition which has excellent activity for exterminating animal parasites for use in a method for exterminating animal parasites is provided.

### DESCRIPTION OF EMBODIMENTS

A composition for exterminating animal parasites according to the present invention includes at least one amide derivative represented by the following Formula (1).

By adopting such a constitution, it becomes possible to show excellent activity for exterminating animal parasites in the case of giving the composition to an animal.

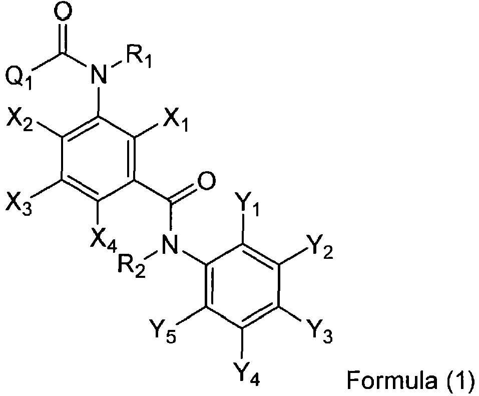

In Formula (1), Q₁ represents a phenyl group or a phenyl group substituted with a halogen atom. X₁ represents a fluorine atom, and X₂, X₃, and X₄ each represent a hydrogen atom. R₁ represents a hydrogen atom or a C1-C3 alkyl group, and R₂ represents a hydrogen atom. Y₁ and Y₅ each independently represent a halogen atom or a C1-C3 haloalkyl group, Y₂ and Y₄ each represent a hydrogen atom, and Y₃ represents a heptafluoroisopropyl group.

The terms used in the formulae including the Formula (1) according to the present invention, have the same meanings as described below in the definitions.

The "halogen atom" represents a fluorine atom, a chlorine atom, a bromine atom, or an iodine atom.

The expression "Ca-Cb (wherein a and b represent an integer of 1 or more)", for example, "C1-C3" means the number of carbon atoms of from 1 to 3, the "C2-C6" means the number of carbon atoms of from 2 to 6.

The "C1-C3 alkyl group" in the present invention represents, for example, a linear or branched alkyl group having from 1 to 3 carbon atoms such as methyl, ethyl, n-propyl, or i-propyl. The "C1-C3 haloalkyl group" represents, for example, a linear or branched alkyl group having from 1 to 3 carbon atoms, that is substituted with one or more halogen atoms which may be the same as or different from each other, such as trifluoromethyl, pentafluoroethyl, heptafluoro-n-propyl, heptafluoro-i-propyl, 2,2-difluoroethyl, 2,2-dichloroethyl, 2,2,2-trifluoroethyl, 2-fluoroethyl, 2-chloroethyl, 2-bromoethyl, 2-iodoethyl, 2,2,2-trichloroethyl, 2,2,2-tribromoethyl, 1,3-difluoro-2-propyl. 1,3-dichloro-2-propyl, 1-chloro-3-fluoro-2-propyl, 1,1,1-trifluoro-2-propyl, 3,3,3-trifluoro-n-propyl 1,1,1,3,3,3-hexafluoro-2-propyl, 1,1,1,3,3,3-hexafluoro-2-chloro-2-propyl, 1,1,1,3,3,3-hexafluoro-2-bromo-2-propyl, 1,1,2,3,3,3-hexafluoro-2-chloro-n-propyl, 1,1,2,3,3,3-hexafluoro-2-bromo-n-propyl, 1,1,2,3,3,3-hexafluoro-1-bromo-2-propyl, 2,2,3,3,3-pentafluoro-n-propyl, 3-fluoro-n-propyl, 3-chloro-n-propyl, or 3-bromo-n-propyl.

The "C1-C6 alkyl group" in the present invention represents, for example, a linear or branched alkyl group having from 1 to 6 carbon atoms such as methyl, ethyl, n-propyl, i-propyl, n-butyl, s-butyl, t-butyl, n-pentyl, 2-pentyl, neopentyl, 4-methyl-2-pentyl, n-hexyl, or 3- methyl-n-pentyl

The "C3-C9 cycloalkyl group" represents, for example, a cycloalkyl group having from 3 to 9 carbon atoms, that has a cyclic structure, such as cyclopropyl, cyclobutyl, cyclopentyl, 2-methylcyclopentyl, 3-methylcyclopentyl, cyclohexyl, 2-methylcyclohexyl, 3-methylcyclohexyl, or 4-methylcyclohexyl.

The "C2-C6 alkenyl group" represents, for example, an alkenyl group having from 2 to 6 carbon atoms, that has a double bond in the carbon chain, such as vinyl, allyl, 2-butenyl, or 3-butenyl

The "C2-C6 alkynyl group" represents, for example, an alkynyl group having from 2 to 6 carbon atoms, that has a triple bond in the carbon chain, such as propargyl, 1-butyn-3-yl, or 1-butyn-3-methyl-3-yl

The amide derivatives represented by Formula (1) according to the present invention may include one or plural chiral carbon atoms or chiral centers in their structural Formulae, and thus two or more optical isomers may exist. However, the present invention includes each of the optical isomers and a mixture thereof at any proportions. Further, the amide derivative represented by Formula (1) according to the present invention may include two or more kinds of geometrical isomers derived from carbon-carbon double bonds in the structural Formulae. The present invention includes each of the geometrical isomers and a mixture thereof at any proportions.

The representative methods for producing the amide derivative according to the present invention are shown below, and the method for producing the amide derivative according to the present invention is not limited to the preparation methods below.

In Formulae shown in the following preparation method, X₁, X₂, X₃, X₄, R₁, R₂, Q₁, Y₁, Y₂, Y₃, Y₄, and Y₅ represent the same definitions as X₁, X₂, X₃, X₄, R₁, R₂, Q₁, Y₁, Y₂, Y₃, Y₄, and Y₅, respectively, in Formula (1). LG represents a functional group having a leaving ability, such as a halogen atom, or a hydroxy group Hal represents a chlorine atom or a bromine atom, and Xa and Xb represent chlorine atoms, bromine atoms, or iodine atoms. R₃ represents a C1-C6 alkyl group, a C3-C9 cycloalkyl group, a C2-C6 alkenyl group, a C2-C6 alkynyl group.

### <Preparation Method 1>

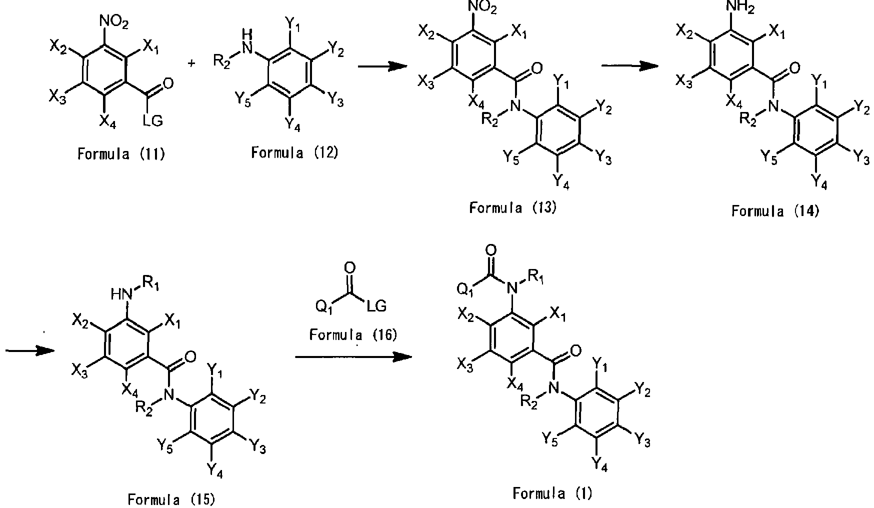

### 1-(i): Formula (11) + Formula (12) → Formula (13)

A nitro aromatic carboxamide derivative represented by Formula (13) can be prepared by reacting a nitro aromatic carboxylic acid derivative having a leaving group represented by Formula (11) with an aromatic amine derivative represented by Formula (12) in a suitable solvent or without a solvent. In the present step, a suitable base can be used.

The solvent may be any of those which do not inhibit the present reaction significantly. Examples thereof may include water and aromatic hydrocarbons such as benzene, toluene, or xylene, halogenated hydrocarbons such as dichloromethane, chloroform, or carbon tetrachloride chained or cyclic ethers such as diethyl ether, dioxane, tetrahydrofuran, or 1,2-dimethoxy ethane esters such as ethyl acetate, or butyl acetate alcohols such as methanol, or ethanol ketones such as acetone, methyl isobutyl ketone, or cyclohexenone amides such as dimethyl formamide, or dimethylacetamide nitriles such as acetonitrile and inert solvents such as 1,3-dimethyl-2-imidazolidinone. These solvents may be used alone or as a mixture of two or more kinds thereof.

Furthermore, examples of the base may include organic bases such as triethylamine, tri-n-butyl amine, pyridine, or 4-dimethylamino pyridine, alkali metal hydroxides such as sodium hydroxide, or potassium hydroxide, carbonates such as sodium hydrogen carbonate, or potassium carbonate phosphates such as dipotassium monohydrogen phosphate, or trisodium phosphate alkali metal hydride salts such as sodium hydride alkali metal alkoxides such as sodium methoxide, or sodium ethoxide, and lithium amides such as lithium diisopropyl amide,

These bases may be appropriately used in an amount in the range from 0.01-fold molar equivalent to 5-fold molar equivalents with respect to the compound represented by Formula (11).

The reaction temperature may be appropriately selected from -20°C to the reflux temperature of the solvent used. Further, the reaction time may be appropriately selected within the range from several minutes to 96 hours.

Among the compounds represented by Formula (11), the aromatic carbonyl halide derivative can be prepared easily by a general method using a halogenating agent from an aromatic carboxylic acid. Examples of the halogenating agent include thionyl chloride, thionyl bromide, phosphorus oxychloride, oxalyl chloride, or phosphorus trichloride.

Meanwhile, it is possible to prepare the compound represented by Formula (13) from the nitro aromatic carboxylic acid derivative and the compound represented by Formula (12) without using a halogenating agent. Examples of the method may include a method described in, for example, Chem. Ber. p. 788 (1970), in which a condensing agent such as N,N'-dicyclohexylcarbodiimide is appropriately used, suitably with the use of an additive such as 1-hydroxybenzotriazole. Other condensing agents that can be used in this case may include 1-ethyl-3-(3-dimethylaminopropyl)carbodiimide, or 1,1'-carbonylbis-1H-imidazole,

Furthermore, examples of other methods for producing the compound represented by Formula (13) may include a mixed acid anhydride method using chloroformic acid esters, and examples thereof include a method described in J. Am. Chem. Soc., p. 5012 (1967), whereby the compound represented by Formula (13) can be prepared. Examples of the chloroformic acid esters used in this case may include isobutyl chloroformate, or isopropyl chloroformate.

In addition to chloroformic acid esters, diethylacetyl chloride, or trimethylacetyl chloride may also be included.

Both the method using a condensing agent and the mixed acid anhydride method are not limited by the solvent, the reaction temperature, and the reaction time according to the literature above. An inert solvent may be used which does not inhibit the progress of the appropriate reaction significantly, and the reaction temperature and the reaction time may also be selected appropriately according to the progress of the reaction.

### 1-(ii): Formula (13) → Formula (14)

An aromatic carboxamide derivative having an amino group represented by Formula (14) can be derived from the aromatic carboxamide derivative having a nitro group represented by Formula (13) by means of a reduction reaction. Examples of such reduction include a method using a hydrogenation reaction and a method using a metal compound (for example, stannous chloride (anhydride), iron powder, or zinc powder).

The reaction of the former method can be carried out in a suitable solvent in the presence of a catalyst at normal pressure or a higher pressure under a hydrogen atmosphere. Examples of the catalyst may include palladium catalysts such as palladium-carbon and the like, nickel catalysts such as Raney-nickel, cobalt catalysts, ruthenium catalysts, rhodium catalysts, or platinum catalysts and examples of the solvent may include water; alcohols such as methanol, or ethanol; aromatic hydrocarbons such as benzene, or toluene; chained or cyclic ethers such as ether, dioxane, or tetrahydrofuran;
and esters such as ethyl acetate. The pressure may be appropriately selected within a range of 0.1 MPa to 10 MPa, the reaction temperature may be appropriately selected within a range of -20°C to the reflux temperature of the solvent used, and the reaction time may be appropriately selected within a range of several minutes to 96 hours, whereby the compound of Formula (14) can be efficiently prepared.

Examples of the latter method include a method using stannous chloride (anhydride) as a metal compound under the conditions described in "Organic Syntheses" Coll. Vol. III, P. 453.

### 1-(iii): Formula (14) → Formula (15)

### (Method A)

A compound represented by Formula (15) can be prepared by reacting the compound represented by Formula (14) with an aldehyde or a ketone in a solvent, and reacting them under a hydrogen atmosphere with the addition of a catalyst.

The solvent may be any of those which do not inhibit the progress of the reaction significantly, and examples thereof may include aliphatic hydrocarbons such as hexane, cyclohexane, or methylcyclohexane; aromatic hydrocarbons such as benzene, xylene, or toluene; halogenated hydrocarbons such as dichloromethane, chloroform, carbon tetrachloride, or 1,2-dichloroethane; ethers such as diethyl ether, or chloroethane; ethers such as diethyl ether, dioxane, tetrahydrofuran, or 1,2-dimethoxy ethane; amides such as dimethyl formamide, dimethylacetamide, and the like, nitriles such as acetonitrile, or propionitrile; ketones such as acetone, methyl isobutyl ketone, cyclohexanone, or methyl ethyl ketone; esters such as ethyl acetate, or butyl acetate; alcohols such as methanol, or ethanol; inert solvents such as 1,3-dimethyl-2-imidazolidinone, sulfolane, or dimethylsulfoxide, and water. These solvents may be used alone or as a mixture of two or more kinds thereof.

Examples of the catalyst may include palladium catalysts such as palladium-carbon, or palladium hydroxide-carbon, nickel catalysts such as Raney-nickel cobalt catalysts, platinum catalysts, ruthenium catalysts, or rhodium catalysts.

Examples of the aldehydes may include formaldehyde, acetaldehyde, propionaldehyde, trifluoroacetaldehyde, difluoroacetaldehyde, fluoroacetaldehyde, chloroacetaldehyde, dichloroacetaldehyde, trichloroacetaldehyde, or bromoacetaldehyde Examples of the ketones may include acetone, perfluoroacetone, or methyl ethyl ketone.

The reaction pressure may be appropriately selected within the range of 1 atm to 100 atm. The reaction temperature may be appropriately selected within the range from -20°C to the reflux temperature of the solvent used. Further, the reaction time may be appropriately selected within the range from several minutes to 96 hours.

### (Method B)

A compound represented by Formula (15) can be prepared by reacting the compound represented by Formula (14) with an aldehyde or a ketone in a solvent, and treating the product with a reducing agent.

The solvent may be any of those which do not inhibit the progress of the reaction significantly, and examples thereof may include aliphatic hydrocarbons such as hexane, cyclohexane, or methylcyclohexane; aromatic hydrocarbons such as benzene, xylene, or toluene; halogenated hydrocarbons such as dichloromethane, chloroform, carbon tetrachloride, or 1,2-dichloroethane; ethers such as diethyl ether, dioxane, tetrahydrofuran, or 1,2-dimethoxy ethane; amides such as dimethyl formamide, or dimethylacetamide; nitriles such as acetonitrile, or propionitrile; ketones such as acetone, methyl isobutyl ketone, cyclohexanone, or methyl ethyl ketone; esters such as ethyl acetate, or butyl acetate; alcohols such as methanol, or ethanol; inert solvents such as 1,3-dimethyl-2-imidazolidinone, sulfolane, or dimethylsulfoxide; and water.. These solvents may be used alone or as a mixture of two or more kinds thereof.

Examples of the reducing agent may include, for example, borohydrides such as sodium borohydride, sodium cyanoborohydride, or sodium triacetate borohydride

Examples of the aldehydes may include formaldehyde, acetaldehyde, propionaldehyde, trifluoroacetaldehyde, difluoroacetaldehyde, fluoroacetaldehyde, chloroacetaldehyde, dichloroacetaldehyde, trichloroacetaldehyde, or bromoacetaldehyde.

Examples of the ketones may include acetone, perfluoroacetone, or methyl ethyl ketone.

The reaction temperature may be appropriately selected within the range from -20°C to the reflux temperature of the solvent used. Further, the reaction time may be appropriately selected within the range from several minutes to 96 hours.

### (Method C)

A compound of Formula (15) can be prepared by reacting the compound represented by Formula (14) with an aldehyde in a solvent or without a solvent.

The solvent may be any of those which do not inhibit the progress of the reaction significantly, and examples thereof may include aliphatic hydrocarbons such as hexane, cyclohexane, or methylcyclohexane; aromatic hydrocarbons such as benzene, xylene, or toluene; halogenated hydrocarbons such as dichloromethane, chloroform, carbon tetrachloride, or 1,2-dichloroethane; ethers such as diethyl ether, dioxane, tetrahydrofuran, or 1,2-dimethoxyethane; amides such as dimethyl formamide, or dimethylacetamide; nitriles such as acetonitrile, or propionitrile; ketones such as acetone, methyl isobutyl ketone, cyclohexanone, or methyl ethyl ketone; esters such as ethyl acetate, or butyl acetate; alcohols such as methanol, or ethanol; inert solvents such as 1,3-dimethyl-2-imidazolidinone, sulfolane, or dimethylsulfoxide; inorganic acids such as sulfuric acid, or hydrochloric acid; organic acids such as formic acid, or acetic acid and water, These solvents may be used alone or as a mixture of two or more kinds thereof.

Examples of the aldehydes may include formaldehyde, acetaldehyde, or propionaldehyde.

The reaction temperature may be appropriately selected within the range from -20°C to the reflux temperature of the solvent used, and the reaction time may be appropriately selected within the range from several minutes to 96 hours.

### 1-(iv): Formula (15) + Formula (16) → Formula (1)

An aromatic carboxamide derivative represented by Formula (1) can be prepared by reacting the aromatic amine derivative represented by Formula (15) with the carboxylic acid derivative or the carbonate ester derivative having a leaving group represented by Formula (16) in a suitable solvent. In the present step, a suitable base or solvent can be used, and as the base or solvent, those exemplified in 1-(i) can be used. Examples of the reaction temperature, or the reaction time may include those exemplified in 1-(i).

In Formula (16), the carbonyl chloride derivative can be prepared easily from a carboxylic acid derivative by a general method using a halogenating agent. The halogenating agent may include those exemplified in 1-(i).

There may be exemplified a method for producing a compound represented by Formula (1) from the carboxylic acid derivative (16) and the compound represented by Formula (15) without the use of a halogenating agent, and the preparation can be conducted according to the method exemplified in 1-(i).

### <Preparation Method 2>

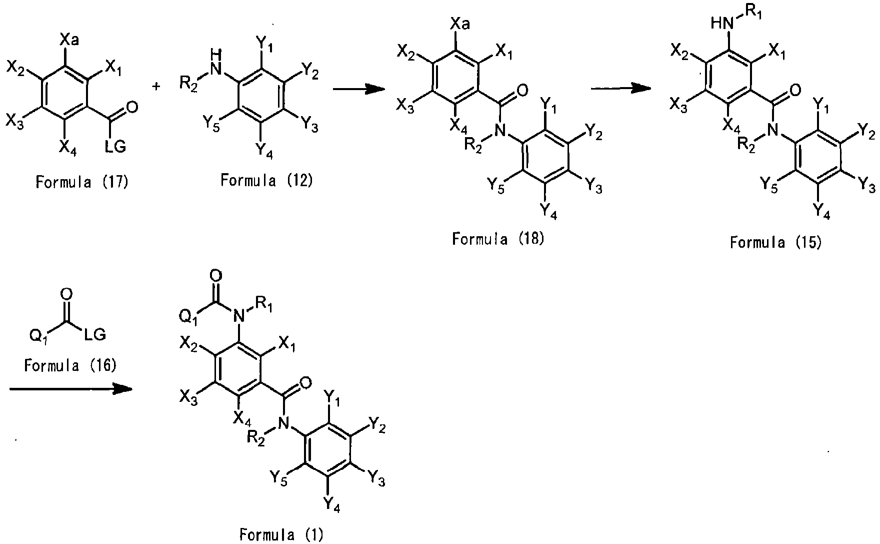

### 2-(i): Formula (17) + Formula (12) → Formula (18)

A compound represented by Formula (18) can be prepared by reacting the compound represented by Formula (17) with a compound represented by Formula (12) under the condition described in 1-(i).

### 2-(ii): Formula (18) → Formula (15)

A compound represented by Formula (15) can be prepared by carrying out an amination reaction using an amination agent such as ammonia, according to the conditions described, for example, in J. Org. Chem. p. 280 (1958). However, the conditions such as a reaction solvent, are not restricted to those described in the literature, and an inert solvent which does not inhibit the proper progress of the reaction significantly may be used. The reaction temperature and reaction time may be suitably selected as the reaction proceeds. Further, examples of the amination agent include methylamine, or ethylamine in addition to ammonia.

### 2-(iii): Formula (15) + Formula (16) → Formula (1)

The compound represented by Formula (1) can be prepared by reacting the compound represented by Formula (15) with a compound represented by Formula (16) according to the conditions described in 1-(i).

### <Preparation Method 3>

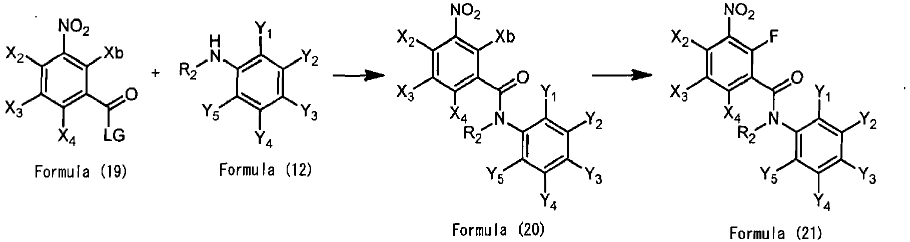

### 3-(i): Formula (19) + Formula (12) → Formula (20)

A compound represented by Formula (20) can be prepared by reacting the compound represented by Formula (19) and the compound represented by Formula (12) according to the conditions described in 1-(i).

### 3-(ii): Formula (20) → Formula (21)

A compound represented by Formula (21) can be prepared by reacting the nitro aromatic carboxamide derivative represented by Formula (20) with a suitable fluorinating agent in a suitable solvent or without a solvent.

The solvent may be any of those which do not inhibit the progress of the reaction significantly, and examples thereof may include aliphatic hydrocarbons such as hexane, cyclohexane, or methylcyclohexane; aromatic hydrocarbons such as benzene, toluene, or xylene; halogenated hydrocarbons such as dichloromethane, chloroform, carbon tetrachloride, or 1,2-dichloroethane; chained or cyclic ethers such as diethyl ether, dioxane, tetrahydrofuran, or 1,2-dimethoxy ethane; esters such as ethyl acetate, or butyl acetate; ketones such as acetone, methyl isobutyl ketone, cyclohexanone, or methyl ethyl ketone; nitriles such as acetonitrile, or propionitrile;
and aprotic polar solvents such as 1,3-dimethyl-2-imidazolidinone, sulfolane, dimethylsulfoxide, N,N-dimethyl formamide, N-methyl pyrrolidone, or N,N-dimethylacetamide.

These solvents may be used alone or as a mixture of two or more kinds thereof.

Examples of the fluorinating agent may include 1,1,2,2-tetrafluoroethyl diethylamine, 2-chloro-1,1,2-trifluoroethyl diethylamine, trifluorodiphenylphospholane, difluorotriphenylphospholane, fluoroformic acid esters, sulfur tetrafluoride, potassium fluoride, potassium hydrogen fluoride, cesium fluoride, rubidium fluoride, sodium fluoride, lithium fluoride, antimony (III) fluoride, antimony (V) fluoride, zinc fluoride, cobalt fluoride, lead fluoride, copper fluoride, mercury (II) fluoride, silver fluoride, silver fluoroborate, thallium (I) fluoride, molybdenum (VI) fluoride, arsenic (III) fluoride, bromine fluoride, selenium tetrafluoride, tris(dimethylamino)sulfonium difluorotrimethylsilicate, sodium hexafluorosilicate, quaternary ammonium fluorides, (2-chloroethyl) diethylamine, diethylaminosulfur trifluoride, morpholinosulfur trifluoride, silicon tetrafluoride, hydrogen fluoride, hydrofluoric acid, hydrogen fluoride-pyridine complex, hydrogen fluoride-triethylamine complex, hydrogen fluoride salts, bis(2-methoxyethyl)amino sulfurtrifluoride, 2,2-difluoro-1,3-dimethyl-2-imidazolidinone, iodine pentafluoride, tris(diethylamino)phosphonium 2,2,3,3,4,4-hexafluorocyclobutanilide, triethylammonium hexafluorocyclobutanilide, or hexafluoropropene. These fluorinating agents may be used alone or as a mixture of two or more kinds thereof.

The fluorinating agent may be appropriately selected and used as a solvent, in the range of 1-fold molar equivalent to 10-fold molar equivalents with respect to the nitro aromatic carboxamide derivative represented by Formula (20).

Additives may be used, and examples thereof may include crown ethers such as 18-crown-6; phase transfer catalysts such as a tetraphenylphosphonium salt;
inorganic salts such as calcium fluoride, or calcium chloride; metal oxides such as mercury oxide; ion exchange resins. These additives may not only be added to the reaction system but also used as a pretreating agent for the fluorinating agent.

The reaction temperature may be appropriately selected within the range from -80°C to the reflux temperature of the solvent used, and the reaction time may be appropriately selected within the range from several minutes to 96 hours.

### <Preparation Method 4>

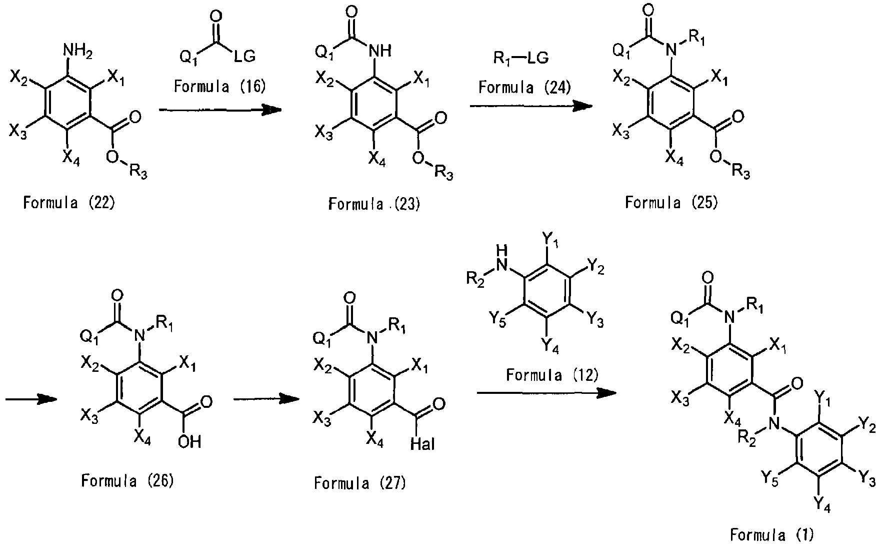

### 4-(i): Formula (22) + Formula (16) → Formula (23)

A carboxylic acid having an acylamino group represented by Formula (23) can be prepared by reacting a compound represented by Formula (22) as a starting material with a compound represented by Formula (16) according to the conditions described in 1-(i).

### 4-(ii): Formula (23) + Formula (24) → Formula (25)

The compound represented by Formula (25) can be prepared by reacting the amide compound represented by Formula (23) with the compound having a leaving group such as a halogen, represented by Formula (24) in a solvent or without a solvent. In the present step, a suitable base or solvent can be used, and as the base or solvent, those exemplified in 1-(i) can be used. Examples of the reaction temperature, or reaction time may include those exemplified in 1-(i).

### 4-(iii): Formula (25) → Formula (26)

The compound represented by Formula (26) can be prepared from the compound represented by Formula (25) through hydrolysis conducted by a general technique or a method using a Pd catalyst. In the hydrolysis, the compound can be obtained by base hydrolysis using one equivalent to 5-fold molar amount of aqueous or alcoholic lithium hydroxide, sodium hydroxide, or potassium hydroxide in a single solvent of methanol, ethanol, tetrahydrofuran, or dioxane, or a combination thereof. Also in a non-aqueous solvent such as toluene and xylene, hydrolysis can be conducted with the combination of a base such as aqueous sodium hydroxide, potassium hydroxide, or lithium hydroxide and a phase-transfer catalyst such as tetrabutylammonium bromide, benzyltriethylammonium chloride, or crown ether. Alternatively, acid hydrolysis can be conducted with inorganic acid such as hydrochloric acid and sulfuric acid, organic acid such as acetic acid and trifluoroacetic acid, or strongly acidic resin.

The reaction temperature may be appropriately selected in the range from -20°C to the reflux temperature of the solvent to be used. And the reaction time may be appropriately selected in the range from a few minutes to 96 hours.

The method using Pd can include, for example, the method described in Tetrahedron Letters p.4371 (1987). The conditions such as the solvent and the reaction temperature are not limited to those described in the reference.

### 4-(iv): Formula (26) -Formula (27)

The compound represented by Formula (27) can be prepared according to the known routine procedure in which the compound represented by Formula (26) is reacted with thionyl chloride, oxalyl chloride, phosgene, phosphorus oxychloride, phosphorus pentachloride, phosphorous trichloride, thionyl bromide, phosphorus tribromide, or diethylaminosulfur trifluoride.

### 4-(v): Formula (27) + Formula (12) →Formula (1)

The compound represented by Formula (1) can be produced by reacting the compound represented by Formula (27) with the compound represented by Formula (12) according to the conditions described in 1-(i).

### 4-(vi): Formula (26) + Formula (12) →Formula (1)

The amide derivative represented by Formula (1) can be produced by reacting the compound represented by Formula (26) with the compound represented by Formula (12) according to the conditions of the method in which a condensing agent is used or the mixed anhydride method both described in 1-(i).

### <Production Method 5>

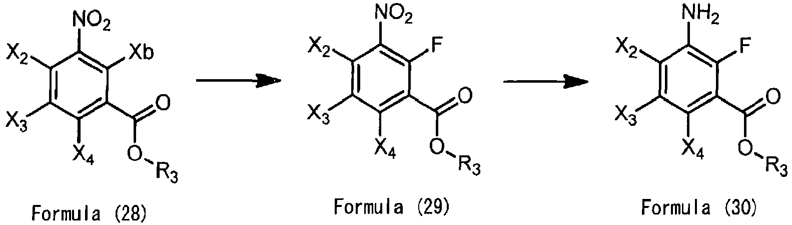

### 5-(i): Formula (28) →Formula (29)

The compound represented by Formula (29) can be produced by fluorinating the compound represented by Formula (28) according to the conditions described in 3-(ii).

### 5-(ii): Formula (29) →Formula (30)

The compound represented by Formula (30) can be produced by reducing the compound represented by Formula (29) according to the conditions described in 1-(ii).

In all of the preparation methods as described above, a desired product may be isolated from the reaction system after the reaction is completed according to a general method, but if required, purification can be carried out by operations such as recrystallization, column chromatography, or distillation. In addition, the desired product can be also provided to the subsequent reaction process without being separated from the reaction system.

Hereinbelow, examples of the representative compounds of the amide derivative represented by Formula (1) as an active ingredient for the composition for exterminating animal parasites according to the present invention will be given in Table 1 and Table 2 below, but the present invention is not limited thereto.

In addition, in the tables, "Me" represents a methyl group, "n-Pr" represents a normal propyl group, "CF3" represents a trifluoromethyl group, "C2F5" represents a pentafluoroethyl group, "n-C3F7" represents a heptafluoronormalpropyl group, "H" represents a hydrogen atom, "F" represents a fluorine atom, "Cl" represents a chlorine atom, "Br" represents a bromine atom, and "I" represents an iodine atom, respectively.

**Table 1(1)**

| | | | | | | | | | | | | |
|---|---|---|---|---|---|---|---|---|---|---|---|---|
| | 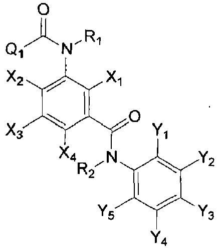 | | | | | | | | | | | |

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-940 | phenyl | H | H | F | H | H | H | F | H | heptafluoroisopropyl | H | F |
| 6-948 | phenyl | H | H | F | H | H | H | Cl | H | heptafluoroisopropyl | H | Cl |
| 6-952 | 2-chlorophenyl | H | H | F | H | H | H | F | H | heptafluoroisopropyl | H | Cl |
| 6-956 | 3-bromophenyl | H | H | F | H | H | H | F | H | heptafluoroisopropyl | H | Br |
| 6-970 | 2,6-difluorophenyl | H | H | F | H | H | H | Cl | H | heptafluoroisopropyl | H | I |
| 6-1104 | phenyl | H | H | F | H | H | H | Br | H | heptafluoroisopropyl | H | Br |
| 6-1105 | 2-fluorophenyl | H | H | F | H | H | H | Br | H | heptafluoroisopropyl | H | Br |
| 6-1106 | 3-fluorophenyl | H | H | F | H | H | H | Br | H | heptafluoroisopropyl | H | Br |
| 6-1107 | 4-fluorophenyl | H | H | F | H | H | H | Br | H | heptafluoroisopropyl | H | Br |
| 6-1126 | 2,6-difluorophenyl | H | H | F | H | H | H | Br | H | heptafluoroisopropyl | H | Br |
| 6-1260 | phenyl | H | H | F | H | H | H | I | H | heptafluoroisopropyl | H | I |
| 6-1261 | 2 -fluorophenyl | H | H | F | H | H | H | I | H | heptafluoroisopropyl | H | I |
| 6-1262 | 3-fluorophenyl | H | H | F | H | H | H | I | H | heptafluoroisopropyl | H | I |
| 6-1263 | 4-fluorophenyl | H | H | F | H | H | H | I | H | heptafluoroisopropyl | H | I |
| 6-1282 | 2,6-difluorophenyl | H | H | F | H | H | H | I | H | heptafluoroisopropyl | H | I |
| 6-1416 | phenyl | H | H | F | H | H | H | F | H | heptafluoroisopropyl | H | CF3 |
| 6-1417 | phenyl | H | H | F | H | H | H | Cl | H | heptafluoroisopropyl | H | CF3 |
| 6-1418 | 2-fluorophenyl | H | H | F | H | H | H | Cl | H | heptafluoroisopropyl | H | CF3 |
| 6-1422 | 3⁻chlorophenyl | H | H | F | H | H | H | Cl | H | heptafluoroisopropyl | H | CF3 |
| 6-1426 | 4-bromophenyl | H | H | F | H | H | H | Cl | H | heptafluoroisopropyl | H | CF3 |
| 6-1439 | 2,6-difluorophenyl | H | H | F | H | H | H | Cl | H | heptafluoroisopropyl | H | CF3 |
| 6-1440 | 3,4-dichlorophenyl | H | H | F | H | H | H | Cl | H | heptafluoroisopropyl | H | CF3 |
| 6-1441 | 2,4-dichlorophenyl | H | H | F | H | H | H | Cl | H | heptafluoroisopropyl | H | C2F5 |
| 6-1442 | 2-chloro-4-fluorophenyl | H | H | F | H | H | H | Cl | H | heptafluoroisopropyl | H | CF3 |
| 6-1445 | 2-bromo-4-chlorophenyl | H | H | F | H | H | H | Cl | H | heptafluoroisopropyl | H | n-C3F7 |
| 6-1446 | 2-bromo-4-fluorophenyl | H | H | F | H | H | H | Cl | H | heptafluoroisopropyl | H | CF3 |
| 6-1574 | phenyl | H | H | F | H | H | H | Br | H | heptafluoroisopropyl | H | CF3 |
| 6-1575 | 2 -fluorophenyl | H | H | F | H | H | H | Br | H | heptafluoroisopropyl | H | CF3 |
| 6-1576 | 3 -fluorophenyl | H | H | F | H | H | H | Br | H | heptafluoroisopropyl | H | CF3 |
| 6-1577 | 4-fluorophenyl | H | H | F | H | H | H | Br | H | heptafluoroisopropyl | H | CF3 |
| 6-1578 | 2-chlorophenyl | H | H | F | H | H | H | Br | H | heptafluoroisopropyl | H | CF3 |
| 6-1579 | 3⁻chlorophenyl | H | H | F | H | H | H | Br | H | heptafluoroisopropyl | H | CF3 |
| 6-1580 | 4-chlorophenyl | H | H | F | H | H | H | Br | H | heptafluoroisopropyl | H | CF3 |
| 6-1581 | 2-bromophenyl | H | H | F | H | H | H | Br | H | heptafluoroisopropyl | H | CF3 |

**Table 1(2)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-1582 | 3-bromophenyl | H | H | F | H | H | H | Br | H | heptafluoroisopropyl | H | CF3 |
| 6-1583 | 4-bromophenyl | H | H | F | H | H | H | Br | H | heptafluoroisopropyl | H | CF3 |
| 6-1584 | 2-iodophenyl | H | H | F | H | H | H | Br | H | heptafluoroisopropyl | H | CF3 |
| 6-1585 | 3-iodophenyl | H | H | F | H | H | H | Br | H | heptafluoroisopropyl | H | CF3 |
| 6-1586 | 4-iodophenyl | H | H | F | H | H | H | Br | H | heptafluoroisopropyl | H | CF3 |
| 6-1596 | 2,6-difluorophenyl | H | H | F | H | H | H | Br | H | heptafluoroisopropyl | H | CF3 |
| 6-1597 | 3,4-dichlorophenyl | H | H | F | H | H | H | Br | H | heptafluoroisopropyl | H | CF3 |
| 6-1598 | 2,4-dichlorophenyl | H | H | F | H | H | H | Br | H | heptafluoroisopropyl | H | CF3 |
| 6-1599 | 2-chloro-4-fluorophenyl | H | H | F | H | H | H | Br | H | heptafluoroisopropyl | H | CF3 |
| 6-1600 | 2-chloro-4,5-difluorophenyl | H | H | F | H | H | H | Br | H | heptafluoroisopropyl | H | CF3 |
| 6-1601 | 4-bromo-2-chlorophenyl | H | H | F | H | H | H | Br | H | heptafluoroisopropyl | H | CF3 |
| 6-1602 | 2-bromo-4-chlorophenyl | H | H | F | H | H | H | Br | H | heptafluoroisopropyl | H | CF3 |
| 6-1603 | 2-bromo-4-fluorophenyl | H | H | F | H | H | H | Br | H | heptafluoroisopropyl | H | CF3 |
| 6-1730 | phenyl | H | H | F | H | H | H | I | H | heptafluoroisopropyl | H | CF3 |
| 6-1731 | 2-fluorophenyl | H | H | F | H | H | H | I | H | heptafluoroisopropyl | H | CF3 |
| 6-1732 | 3-fluorophenyl | H | H | F | H | H | H | I | H | heptafluoroisopropyl | H | CF3 |
| 6-1733 | 4-fluorophenyl | H | H | F | H | H | H | I | H | heptafluoroisopropyl | H | CF3 |
| 6-1734 | 2-chlorophenyl | H | H | F | H | H | H | I | H | heptafluoroisopropyl | H | CF3 |
| 6-1735 | 3⁻chlorophenyl | H | H | F | H | H | H | I | H | heptafluoroisopropyl | H | CF3 |
| 6-1736 | 4-chlorophenyl | H | H | F | H | H | H | I | H | heptafluoroisopropyl | H | CF3 |
| 6-1737 | 2-bromophenyl | H | H | F | H | H | H | I | H | heptafluoroisopropyl | H | CF3 |
| 6-1738 | 3-bromophenyl | H | H | F | H | H | H | I | H | heptafluoroisopropyl | H | CF3 |
| 6-1739 | 4-bromophenyl | H | H | F | H | H | H | I | H | heptafluoroisopropyl | H | CF3 |
| 6-1740 | 2-iodophenyl | H | H | F | H | H | H | I | H | heptafluoroisopropyl | H | CF3 |
| 6-1741 | 3-iodophenyl | H | H | F | H | H | H | I | H | heptafluoroisopropyl | H | CF3 |
| 6-1742 | 4-iodophenyl | H | H | F | H | H | H | I | H | heptafluoroisopropyl | H | CF3 |
| 6-1752 | 2,6-difluorophenyl | H | H | F | H | H | H | I | H | heptafluoroisopropyl | H | CF3 |
| 6-1753 | 3,4-dichlorophenyl | H | H | F | H | H | H | I | H | heptafluoroisopropyl | H | CF3 |
| 6-1754 | 2.4-dichlorophenyl | H | H | F | H | H | H | I | H | heptafluoroisopropyl | H | CF3 |
| 6-1755 | 2-chloro-4-fluorophenyl | H | H | F | H | H | H | I | H | heptafluoroisopropyl | H | CF3 |
| 6-1756 | 2-chloro-4,5-difluorophenyl | H | H | F | H | H | H | I | H | heptafluoroisopropyl | H | CF3 |
| 6-1757 | 4-bromo-2-chlorophenyl | H | H | F | H | H | H | I | H | heptafluoroisopropyl | H | CF3 |
| 6-1758 | 2-bromo-4-chlorophenyl | H | H | F | H | H | H | I | H | heptafluoroisopropyl | H | CF3 |
| 6-1759 | 2-bromo-4-fluorophenyl | H | H | F | H | H | H | I | H | heptafluoroisopropyl | H | CF3 |
| 6-2110 | phenyl | H | H | F | H | H | H | CF3 | H | heptafluoroisopropyl | H | CF3 |
| 6-5902 | phenyl | H | H | F | H | H | H | Br | H | heptafluoroisopropyl | H | Cl |
| 6-5910 | phenyl | H | H | F | H | H | H | Br | H | heptafluoroisopropyl | H | C2F5 |

**Table 2(1)**

| | | | | | | | | | | | | |
|---|---|---|---|---|---|---|---|---|---|---|---|---|
| | 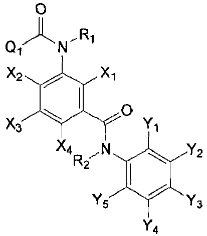 | | | | | | | | | | | |

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-940 | phenyl | Me | H | F | H | H | H | F | H | heptafluoroisopropyl | H | F |
| 7-948 | phenyl | Me | H | F | H | H | H | Cl | H | heptafluoroisopropyl | H | Cl |
| 7-1104 | phenyl | Me | H | F | H | H | H | Br | H | heptafluoroisopropyl | H | Br |
| 7-1105 | 2-fluorophenyl | Me | H | F | H | H | H | Br | H | heptafluoroisopropyl | H | Br |
| 7-1106 | 3-fluorophenyl | Me | H | F | H | H | H | Br | H | heptafluoroisopropyl | H | Br |
| 7-1107 | 4 -fluorophenyl | Me | H | F | H | H | H | Br | H | heptafluoroisopropyl | H | Br |
| 7-1126 | 2,6-difluorophenyl | Me | H | F | H | H | H | Br | H | heptafluoroisopropyl | H | Br |
| 7-1260 | phenyl | Me | H | F | H | H | H | I | H | heptafluoroisopropyl | H | I |
| 7-1261 | 2 -fluorophenyl | Me | H | F | H | H | H | J | H | heptafluoroisopropyl | H | I |
| 7-1262 | 3-fluorophenyl | Me | H | F | H | H | H | I | H | heptafluoroisopropyl | H | I |
| 7-1263 | 4-fluorophenyl | Me | H | F | H | H | H | I | H | heptafluoroisopropyl | H | I |
| 7-1282 | 2,6-difluorophenyl | Me | H | F | H | H | H | I | H | heptafluoroisopropyl | H | I |
| 7-1416 | phenyl | Me | H | F | H | H | H | F | H | heptafluoroisopropyl | H | CF3 |
| 7-1417 | phenyl | Me | H | F | H | H | H | Cl | H | heptafluoroisopropyl | H | CF3 |
| 7-1441 | 2.4-dichlorophenyl | Me | H | F | H | H | H | Cl | H | heptafluoroisopropyl | H | C2F5 |
| 7-1442 | 2-chloro-4-fluarophenyl | n-Pr | H | F | H | H | H | Cl | H | heptafluoroisopropyl | H | CF3 |
| 7-1445 | 2-bromo-4-chlorophenyl | Me | H | F | H | H | H | Cl | H | heptafluoroisopropyl | H | n-C3F7 |
| 7-1574 | phenyl | Me | H | F | H | H | H | Br | H | heptafluoroisopropyl | H | CF3 |
| 7-1575 | 2-fluorophenyl | Me | H | F | H | H | H | Br | H | heptafluoroisopropyl | H | CF3 |
| 7-1576 | 3-fluorophenyl | Me | H | F | H | H | H | Br | H | heptafluoroisopropyl | H | CF3 |
| 7-1577 | 4-fluorophenyl | Me | H | F | H | H | H | Br | H | heptafluoroisopropyl | H | CF3 |
| 7-1578 | 2-chlorophenyl | Me | H | F | H | H | H | Br | H | heptafluoroisopropyl | H | CF3 |
| 7-1579 | 3-chlorophenyl | Me | H | F | H | H | H | Br | H | heptafluoroisopropyl | H | CF3 |
| 7-1580 | 4-chlorophenyl | Me | H | F | H | H | H | Br | H | heptafluoroisopropyl | H | CF3 |
| 7-1581 | 2-bromophenyl | Me | H | F | H | H | H | Br | H | heptafluoroisopropyl | H | CF3 |
| 7-1582 | 3-bromophenyl | Me | H | F | H | H | H | Br | H | heptafluoroisopropyl | H | CF3 |
| 7-1583 | 4-bromophenyl | Me | H | F | H | H | H | Br | H | heptafluoroisopropyl | H | CF3 |
| 7-1584 | 2-iodophenyl | Me | H | F | H | H | H | Br | H | heptafluoroisopropyl | H | CF3 |
| 7-1585 | 3-iodophenyl | Me | H | F | H | H | H | Br | H | heptafluoroisopropyl | H | CF3 |
| 7-1586 | 4-iodophenyl | Me | H | F | H | H | H | Br | H | heptafluoroisopropyl | H | CF3 |
| 7-1596 | 2,6- difluorophenyl | Me | H | F | H | H | H | Br | H | heptafluoroisopropyl | H | CF3 |
| 7-1597 | 3,4- dichlorophenyl | Me | H | F | H | H | H | Br | H | heptafluoroisopropyl | H | CF3 |
| 7-1598 | 2,4-dichlorophenyl | Me | H | F | H | H | H | Br | H | heptafluoroisopropyl | H | CF3 |

**Table 2(2)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-1599 | 2-chloro-4-fluorophenyl | Me | H | F | H | H | H | Br | H | heptafluoroisopropyl | H | CF3 |
| 7-1600 | 2-chloro-4,5-difluorophenyl | Me | H | F | H | H | H | Br | H | heptafluoroisopropyl | H | CF3 |
| 7-1601 | 4-bromo-2-chlorophenyl | Me | H | F | H | H | H | Br | H | heptafluoroisopropyl | H | CF3 |
| 7-1602 | 2-bromo-4-chlorophenyl | Me | H | F | H | H | H | Br | H | heptafluoroisopropyl | H | CF3 |
| 7-1603 | 2-bromo-4-fluorophenyl | Me | H | F | H | H | H | Br | H | heptafluoroisopropyl | H | CF3 |
| 7-1730 | phenyl | Me | H | F | H | H | H | I | H | heptafluoroisopropyl | H | CF3 |
| 7-1731 | 2-fluorophenyl | Me | H | F | H | H | H | I | H | heptafluoroisopropyl | H | CF3 |
| 7-1732 | 3-fluorophenyl | Me | H | F | H | H | H | I | H | heptafluoroisopropyl | H | CF3 |
| 7-1733 | 4-fluorophenyl | Me | H | F | H | H | H | I | H | heptafluoroisopropyl | H | CF3 |
| 7-1734 | 2-chlorophenyl | Me | H | F | H | H | H | I | H | heptafluoroisopropyl | H | CF3 |
| 7-1735 | 3⁻chlorophenyl | Me | H | F | H | H | H | I | H | heptafluoroisopropyl | H | CF3 |
| 7-1736 | 4-chlorophenyl | Me | H | F | H | H | H | I | H | heptafluoroisopropyl | H | CF3 |
| 7-1737 | 2-bromophenyl | Me | H | F | H | H | H | I | H | heptafluoroisopropyl | H | CF3 |
| 7-1738 | 3-bromophenyl | Me | H | F | H | H | H | I | H | heptafluoroisopropyl | H | CF3 |
| ^{.} 7-1739 | 4-bromophenyl | Me | H | F | H | H | H | I | H | heptafluoroisopropyl | H | CF3 |
| 7-1740 | 2-iodophenyl | Me | H | F | H | H | H | I | H | heptafluoroisopropyl | H | CF3 |
| 7-1741 | 3-iodophenyl | Me | H | F | H | H | H | I | H | heptafluoroisopropyl | H | CF3 |
| 7-1742 | 4-iodophenyl | Me | H | F | H | H | H | I | H | heptafluoroisopropyl | H | CF3 |
| 7-1752 | 2.6-difluorophenyl | Me | H | F | H | H | H | I | H | heptafluoroisopropyl | H | CF3 |
| 7-1753 | 3,4-dichlorophenyl | Me | H | F | H | H | H | I | H | heptafluoroisopropyl | H | CF3 |
| 7-1754 | 2,4-dichlorophenyl | Me | H | F | H | H | H | I | H | heptafluoroisopropyl | H | CF3 |
| 7-1755 | 2-chloro-4-fluorophenyl | Me | H | F | H | H | H | I | H | heptafluoroisopropyl | H | CF3 |
| 7-1756 | 2-chloro-4,5-difluorophenyl | Me | H | F | H | H | H | I | H | heptafluoroisopropyl | H | CF3 |
| 7-1757 | 4-bromo-2-chlorophenyl | Me | H | F | H | H | H | I | H | heptafluoroisopropyl | H | CF3 |
| 7-1758 | 2-broma-4-chlorophenyl | Me | H | F | H | H | H | I | H | heptafluoroisopropyl | H | CF3 |
| 7-1759 | 2-bromo-4-fluorophenyl | Me | H | F | H | H | H | I | H | heptafluoroisopropyl | H | CF3 |
| 7-2110 | phenyl | Me | H | F | H | H | H | CF3 | H | heptafluoroisopropyl | H | CF3 |
| 7-5902 | phenyl | Me | H | F | H | H | H | I | H | heptafluoroisopropyl | H | Br |

Hereinbelow, Table 3 shows the physical properties of the representative compounds of the amide derivative according to the present invention. The ¹H-NMR chemical shift values shown therein are based on tetramethylsilane as an internal standard substance unless specified otherwise.

**Table 3(1)**

| compound | ¹H-NMR (CDCl₃, ppm) |
|---|---|
| 6-1104 | *δ* 7.40 (1H, t, J =7.8Hz), 7.53-7.64 (3H, m), 7.89 (2H, s), 7.90-7.95 (3H, m), 8.11-8.14 (2H, m), 8.69-8.70 (1H, m). |
| 6-1105 | *δ* 7.22-7.26 (1H, m), 7.35-7.41 (2H, m), 7.58 (1H, d, J =8.0Hz), 7.88-7.92 (3H, m), 8.15-8.25 (2H, m), 8.74-8.75 (2H, m). |
| 6-1106 | *δ* 7.35-7.34 (1H, m), 7.417.42 (1H, m), 7.53-7.54 (1H, m), 7.67-7.68 (2H, m), 7.89-7.90 (3H, m), 8.09-8.10 (2H, m), 8.66-8.68 (1H, m). |
| 6-1107 | *δ* 7.21-7.26 (2H, m), 7.39-7.41 (1H, m), 7.89-7.96 (4H, m), 8.05-8.13 (3H, m), 8.70-8.72 (1H, m). |
| 6-1126 | *δ* 7.05-7.09 (2H, m), 7.38-7.42 (1H, m), 7.49-7.50 (1H, m), 7.88-7.99 (4H, m), 8.09-8.12 (1H, m), 8.71-8.72 (1H, m). |
| 6-1260 | *δ* 7.39 (1H, t, J = 7.8Hz), 7.52-7.57 (4H, m), 7.60-7.63 (2H, m), 7.93-7.94 (4H, m), 8.70 (1H, t, J =6.3Hz). |
| 6-1261 | *δ* 7.22-7.28 (1H, m), 7.35-7.42 (2H, m), 7.59-7.61 (1H, m), 7.94-7.95 (1H, m), 8.12 (2H, s), 8.15-8.25 (2H, m), 8.78 (1H, t, J =1.5Hz), 9.00 (1H, d, J =7.8Hz). |
| 6-1262 | *δ* 7.30-7.33(1 H, m), 7.40-7.44(1 H, m), 7.52-7.55(2H, m), 7.64-7.70(3H, m), 7.95-7.96(1 H, m), 8.09-8.13(2H, m), 8.67-8.68(1H, m). |
| 6-1263 | *δ* 7.20-7.26 (3H, m), 7.38-7.42 (1H, m), 7.91-7.98 (3H, m), 8.07-8.12 (3H, m), 8.63-8.67 (1H, m). |
| 6-1282 | *δ* 7.06 (2H, t, J =8.3Hz), 7.38-7.42 (1H, m), 7.47-7.52 (1H, m), 7.93-7.97 (1H, m), 8.01 (1H, s), 8.11-8.13 (3H, m), 8.68-8.72 (1H, m). |
| 6-1574 | *δ* 7.37-7.41 (1H, m), 7.53-7.64(3H, m), 7.86-7.94(4H, m), 8.13-8.22(3H, m), 8.67-8.72(1 H, m). |
| 6-1575 | *δ* 7.22-7.27 (1H, m), 7.35-7.41 (2H, m), 7.57-7.60 (1H, m), 7.88-7.93 (2H, m), 8.16 (1H, s), 8.20-8.25 (2H, m), 8.74-8.75 (1H, m), 8.77-9.00 (1H, m). |
| 6-1576 | *δ* 7.27-7.37 (2H, m), 7.48-7.53 (1H, m), 7.72-7.80 (3H, m), 7.92 (1H, s), 8.15 (1H, s), 8.27-8.31 (1H, m), 9.16 (1H, s), 9.35 (1H, d, J =7.3Hz). |
| 6-1577 | *δ* 7.20-7.23 (3H, m), 7.24-7.26 (1H, m), 7.39-7.40 (3H, m), 7.86-7.97 (1H, m), 8.06 (1H, d, J =2.4Hz), 8.16-8.20 (1H, m), 8.62-8.67 (1H, m). |
| 6-1596 | *δ* 7.00-7.09 (2H, m), 7.40-7.41 (1H, m), 7.46-7.53 (1H, m), 7.89-7.92 (2H, m), 8.00 (1H, s), 8.16-8.19 (2H, m), 8.71-8.72 (1H, m). |
| 6-1730 | *δ* 7.40 (1H, t, J =7.8Hz), 7.53-7.64 (3H, m), 7.87-7.95 (4H, m), 8.14 (1H, s), 8.22 (1H, d, J =12.7Hz), 8.37 (1H, s), 8.71-8.72 (1H, m). |

**Table 3(2)**

| compound number | ¹H-NMR (CDCl₃, ppm) |
|---|---|
| 6-1731 | *δ* 7.22-7.25(1 H, m), 7.35-7.37(2H, m), 7.58-7.60(1 H, m), 7.90(1 H, t, J =8.6Hz), 7.96(1 H, s), 8.22(1 H, t, J =8.8Hz), 8.29-8.32(1 H, d, J =12.4Hz), 8.37(1 H, s), 8.73(1 H, m), 8.94(1 H, s). |
| 6-1732 | *δ* 7.32-7.33(1H, m), 7.37(1H, t, J =8.0Hz), 7.52-7.54(1H, m), 7.64-7.70(2H, m), 7.90(1H, t, J =6.4Hz), 7.96(1H, s), 8.10(1H, s), 8.23(1H, d, J =12.0Hz), 8.37(1H, s), 8.65(1H, t, J =8.0Hz). |
| 6-1733 | *δ* 7.19-7.25(2H, m), 7.35(1 H, t, J =7.8Hz), 7.87-7.97(4H, m), 8.08(1 H, s), 8.25(1H, d, J =12.0Hz), 8.37(1H, s), 8.65(1 H, t, J =8.0Hz). |
| 6-1734 | *δ* 7.43-7.52(5H, m), 7.87-7.95(3H, m), 8.22(1 H, d, J =10.0Hz), 8.35(1 H, d, J =8.2Hz), 8.74(1 H, t, J =8.4Hz). |
| 6-2110 | *δ* 7.36-7.40 (1H, m), 7.53-7.64 (3H, m), 7.84-7.97 (1H, m), 7.92-7.94 (2H, m), 8.04-8.07 (1H, m), 8.08-8.13 (1H, m), 8.20 (2H, s), 8.68-8.72 (1H, m). |
| 6-5902 | (DMSO-d₆) *δ* 7.39(1H, t, J = 7.8Hz), 7.52-7.64(4H, m), 7.81(1H, t, J = 6.8Hz), 7.95(1H, s), 7.98-8.01(3H, m), 10.29(1H, s), 10.68(1H, s). |
| 6-5910 | *δ* 7.39(1H, t, J = 8.3Hz), 7.53-7.64(3H, m), 7.88-7.94(4H, m), 8.13(1H, broad-s), 8.19(1H, broad-s), 8.24(1H, d, J = 13.2Hz), 8.70-8.72(1H, m). |
| 7-948 | *δ* 3.49 (3H, s), 7.23-7.52 (8H, m), 7.66 (2H, s), 8.00 (1H, t, J =6.8Hz). |
| 7-1104 | *δ* 3.51 (3H, s), 7.22-7.44 (7H, m), 7.86 (2H, s), 8.00-8.03 (2H, m). |
| 7-1105 | *δ* 3.52 (3H,s), 6.82 (1H, t, J =8.8Hz), 7.06-7.08 (1H, m), 7.18-7.24 (2H, m), 7.40-7.44 (2H, m), 7.87 (3H, s), 8.01-8.05 (1H, m). |
| 7-1106 | *δ* 3.50 (3H,s), 7.00-7.23 (4H, m), 7.29-7.31 (1H, m), 7.45 (1H, s), 7.87 (3H, s), 8.03-8.07 (1H, m). |
| 7-1107 | *δ* 3.49 (3H,s), 6.91-6.93 (2H, m), 7.28-7.44 (4H, m), 7.86 (2H, s), 8.00-8.10 (2H, m). |
| 7-1126 | *δ* 3.49 (3H,s), 6.72-6.76 (2H, m), 7.16-7.23 (2H. m), 7.43-7.50(1H, m), 7.88 (2H, s), 8.02-8.06 (1H, m), 8.13-8.17(1H, m). |
| 7-1260 | *δ* 4.09 (3H, s), 7.21-7.49 (7H, m), 7.99-8.08 (4H, m). |
| 7-1261 | *δ* 3.53 (3H, s), 6.79-6.83 (1H, m), 7.03-7.07 (1H, m), 7.19-7.23 (2H, m), 7.42-7.43 (2H, m), 8.01-8.10 (4H. m). |

**Table 3(3)**

| compound number | ¹H-NMR (CDCl₃, ppm) |
|---|---|
| 7-1417 | *δ* 3.49 (3H, s), 7.23-7.26 (3H, m), 7.27-7.33 (3H, m), 7.52-7.53 (1H, m), 7.85 (1H, s), 7.96-8.06 (3H, m). |
| 7-1574 | *δ* 3.50(3H, s), 6.99-7.33(6H, m), 7.43-7.45(1 H, m), 7.90(1 H, s), 7.97-8.06(2H, m), 8.13(1 H, s). |
| 7-1575 | *δ* 3.52 (3H, s), 6.82-6.83 (1H, m), 7.06-7.07 (1H, m), 7.19-7.26 (2H, m), 7.39-7.46 (2H, m), 7.91 (1H, s), 7.99-8.01 (1H, m), 8.07-8.14 (2H, m). |
| 7-1576 | *δ* 3.50 (3H, s), 7.01-7.17 (4H, m), 7.27-7.31 (1H, m), 7.46-7.52 (1H, m), 7.91 (1H, s), 8.01-8.05 (2H, m), 8.13 (1H, s). |
| 7-1577 | *δ* 3.50 (3H, s), 6.90-6.94 (2H, m), 7.26-7.35 (3H, m), 7.45-7.46 (1H, m), 7.90 (1H, s), 8.00-8.07 (2H, m), 8.13 (1H, s). |
| 7-1596 | *δ* 3.54 (3H, s), 6.75 (2H, broad-s), 7.17-7.26 (2H, m), 7.50-7.51 (1H, m), 7.92 (1H, s), 8.01-8.05 (1H, m), 8.14-8.20 (2H, m). |
| 7-1730 | *δ* 3.51 (3H, s), 7.21-7.23 (2H, m), 7.27-7.33 (4H, m), 7.44-7.46 (1H, m), 7.92 (1H, s), 8.00 (1H, t, J =6.3Hz), 8.08-8.09 (1H, m), 8.33 (1H, s). |
| 7-1731 | *δ* 3.51(3H, s), 6.79-6.83(1H, m), 7.05(1H, t, J =7.6Hz), 7.18-7.46(4H, m), 7.94-8.00(2H, m), 8.20(1H, d, J =12.4Hz), 8.34(1H, s). |
| 7-1732 | *δ* 3.50(3H, s), 7.00-7.18(4H, m), 7.27-7.31(1H, m), 7.45-7.48(1H, m), 7.93(1H, s), 8.01-8.03(1H, m), 8.12(1H, broad-s), 8.34(1H, s). |
| 7-1733 | *δ* 3.50(3H, s), 6.91(2H, s), 6.93-7.35(3H, m), 7.47(1H, t, J =7.0Hz), 7.93(1H, s), 8.01-8.10(1H, m), 8.13(1H, broad-s), 8.34(1 H, s). |
| 7-1752 | *δ* 3.54(3H, s), 6.74(2H, s), 7.16-7.24(2H, m), 7.50(1H, t, J= 7.4Hz), 7.94(1H, s), 8.01-8.05(1H, m), 8.25(1H, broad-s), 8.35(1H, s). |
| 7-5902 | *δ* 3.51 (3H, s), 7.00-7.52 (7H, m), 7.88 (1H, d, J =1.5Hz), 8.01-8.06 (3H, m). |

In particular, for example, animal parasites that can be exterminated by the composition for exterminating animal parasites according to the invention include the followings, although the invention is not limited thereto.

The ectoparasites include Siphonaptera pests such as *Ctenocephalides felis*, *Ctenocephalides canis*, *Xenopsylla cheopis*, *Echidnophaga gallinacea),* and *Pulex irritans*; acarine pests such as *Haemaphysalis longicornis Haemaphysalis japonica*, *Rhipicephalus sanguineus, Boophilus microplus, Dermacentor recticulatus*, *Dermacentor taiwanensis*, *Haemaphysalis flava*, *Ixodes ovatus*, *Ixodes persulcatus*, *Amblyomma americanum*, *Amblyomma maculatum*, *Dermacentor andersoni*, *Dermacentor occidentalis*, *Dermacentor variabilis*, *Haemaphysalis campanulata*, *Haemaphysalis megaspinosa*, *Ixodes nipponensis*, *Ixodes pacifcus*, *Ixodes ricinus*, and *Ixodes scapularisand*;
dipterous pests such as *Musca hervei*, *Musca bezzii*, *Haematobia irritans*, *Simulium iwatens*, *Culicoides oxystoma*, *Tabanus chrysurus*, *Culex pipiens*, and *Aedes albopictus*;
Phthiraptera pests such as *Haematopinus eurysternus* and *Damalinia ovis*; and the like.

The endoparasites include:
Protozoa such as *Rhizopoda* including *Endamoeba histolytica*, *Mastigophora* including *Leishmania* and *Trichomonas*, *Sporozoea* including *Plasmodium* and *Toxoplasma*, and *Ciliophora* including *Balantidium coli*;
*helminths* such as *Nematoda* including *Ascaris lumbricoides* and *Ancylostoma*, *Acannthocephala* including *Macracanthorhynchus hirudinaceus*, *Nematomorpha* including *Paragordius tricuspidatus*, *Trematoda* including *Clonorchis sinensis*, and *Cestoda* including *Taenia saginata*;
*nematodes* such as *Ascaris*, *Toxocara*, *Toxascaris*, *Parascaris*, *Ascaridia*, *Heterakis*, *Oxyuris*, *Capillaria*, *Trichinella*, *Strongylus*, *Triodontophorus*, *Trichonema*, *Stephanurus*, *Desophagostomum*, *Chabertia*, *Syngamus*, *Ancylostoma*, *Uncinaria*, *Necator*, *Bunostomum*, *Trichostrongylus*, *Cooperia*, *Nematodirus*, *Haemonchus*, *Ostertagia*, *Dictyocaulus*, *Metastrongylus*, *Dirofilaria*, *Parafilaria*, *Setaria*, *Onchocerca*, *Habronema*, *Arduenna*, and *Acuaria*;
cestodes such as *Diphyllobothrium*, *Anoplocephara*, *Moniezia*, *Dipylidium*, *Taenia*, *Dithyridium*, *Raillietina*, and *Echinococcus*;
flukes such as *Schistosoma*, *Paramphistomum*, and *Fasciola.*

In the present invention, said animal parasites are preferably ectoparasites from the viewpoint of parasiticidal activity, and they are preferably at least one of Siphonaptera pests (especially preferably *Ctenocephalides felis*) and Acarina pests (especially preferably *Haemaphysalis longicornis*, *Rhipicephalus sanguineus*, and *Boophilus microplus*).

The animals to which the composition for exterminating animal parasites according to the invention can be applied include domestic animals such as horses, cows, pigs, sheep, goats, rabbits, camels, buffalos, deer, minks, and chinchillas; fowls such as chickens, ducks, geese, and turkeys; pets such as dogs, cats, small birds, and monkeys; laboratory animals such as rats, mice, golden hamsters, and guinea pigs; (it is preferable to exclude humans) although the invention is not limited thereto.

The composition for exterminating animal parasites according to the invention can be used as a parasiticide by any of the methods normally used, with no particular restriction.

In particular, for example, the composition may be dissolved, suspended, mixed, impregnated, adsorbed, or adhered on suitable solid and/or liquid carriers according to a formulation generally used, and, if required, together with adjuvant in a suitable proportion. And the composition may be prepared into an appropriate form in accordance with the intended use.

The solid or liquid carrier for use in the invention may be those normally used in agents for animals. From the viewpoint of easiness of treatment on the target animals, it is preferable to use a liquid carrier. The liquid carrier includes, for example, alcohol such as methyl alcohol, ethyl alcohol, isopropyl alcohol, tertiary butyl alcohol, and benzyl alcohol; propylene carbonate; N-methyl-2-pyrrolidone; and water. As the adjuvant, surfactant, antioxidant, or emulsifier can be used. The adjuvant can include, for example, surfactant such as polyoxyethylene alkylaryl ether, polyoxyethylene sorbitan monolaurate, alkyl allyl sorbitan monolaurate, alkylbenzenesulfonate, alkylnaphthalene sulfonic acid, ligninsulfonic acid salts, higher alcohol sulfate salts, glycol monoalkyl ethers, and glycols; emulsifier such as sorbitan monooleate, sorbitan monolaurate, caprylic acid monoglyceride, capric acid monoglyceride, isostearic acid monoglyceride, and propylene glycol monocaprylate; and antioxidant such as BHA and BHT.

The composition for exterminating animal parasites according to the invention may be administered orally or parenterally to an animal.

If the composition for exterminating animal parasites according to the invention is administered orally, the composition may be in the form of capsules, tablets, pills, powders, granules, fine granules, syrups, enteric agents, suspensions, paste, or beverage or feeds mixed with the drug.

If the composition for exterminating animal parasites according to the invention is administered parenterally, the composition may be in the form of injectables, drips, suppositories, emulsions, suspensions, drops, ointments, creams, solutions, lotions, sprays, aerosols, cataplasms, and tapes.

The method for administration includes the spot-on method in which drops are dropped on the skin of the back shoulder region of the target animal to exterminate ectoparasites; local methods such as the pour-on method in which a liquid agent is applied along the back center line of the target animal to allow the applied agent to diffuse on the body surface, resulting in control of ectoparasites; the methods in which the agent is released from a collar containing the agent; the methods in which a liquid agent, or ointment is directly applied to the body surface; the methods in which an aerosol is applied with a spray; the methods in which an injectable is injected intramuscularly, or subcutaneously; and rectal administration with a suppository.

In addition to extermination of endoparasites and exoparasites, the composition for exterminating animal parasites according to the invention also can prophylactically prevent parasitic infections by applying it to the environments which are to be the infection routes. For example, the composition can prevent soil infections from soils of upland fields and parks; percutaneous infections from aqueous systems such as river, lake, wetland, and paddy field; oral infections from excrements of animals such as dogs and cats; and oral infections from raw meats of sea water fish, fresh water fish, Crustaceae, shellfish, or domestic animals;
and infections from mosquitoes, horseflies, flies, cockroaches, ticks, fleas, lice, assassin bugs, or chiggers.

If the composition for exterminating animal parasites according to the invention is used to exterminate parasites in the animals that are mammals or birds, the optimal dosage varies depending on whether it is used for therapeutic purposes or for preventive purposes, and also varies with the type of infected parasites, the type and extent of infection, or dosage form. But generally, in case of oral administration, the dosage is in the range from about 0.0001 to 10,000 mg per kilogram of body weight per day. In case of parenteral administration, the dosage is in the range from about 0.0001 to 10,000 mg per kilogram of body weight per day, and the composition is administered in single or in divided doses.

The concentration of the active ingredient in the composition for exterminating animal parasites according to the invention is typically from 0.0001 to 100% by weight, preferably from 0.001 to 99% by weight, and more preferably from 0.005 to 80% by weight. In general, the parasiticides may be provided as a high-concentrated composition to be diluted to the appropriate concentration prior to use.

In addition to the amide derivatives represented by Formula (1) according to the invention, the composition for exterminating animal parasites according to the invention can further contain other insecticidal components that are generally known. The other insecticidal components can include, for example, pyrethroid compounds such as permethrin, d-phenothrin, allethrin, pyrethrum, prallethrin, cyphenothrin, cyfluthrin, fenvalerate, fenpropathrin, transfluthrin, metofluthrin, resmethrin, cypermethrin, alpha-cypermethrin, bifenthrin, deltamethrin, lambda-cyhalothrin, d,d-trans-cyphenothrin, tetramethrin, and ethofenprox; organic phosphorus compounds such as dichlorvos, tetrachlorvinphos, fenthion, chlorpyrifos, chlorpyrifos-methyl, malathion, pirimiphos-methyl, fenitrothion, and diazinon; N-phenylpyrazole compounds such as fipronil; carbamate compounds such as propoxur, carbaryl, metoxadiazone, and fenocarb; neonicotinoid compounds such as imidacloprid, clothianidin, thiamethoxam, acetamiprid, nitenpyram, and dinotefuran; growth inhibitors for insect such as methoprene, pyriproxyfen, lufenuron, fenoxycarb, triflumuron, and chromafenozide; milbemycin oxime; milbemectin; lepimectin; abamectin; ivermectin; selamectin; spinosad; or rotenone.

### EXAMPLES

Representative Examples of the present invention will be described with reference to the following Examples, but the present invention is not limited thereto. In the present Examples, DMF means N,N-dimethyl formamide, THF means tetrahydrofuran, IPE means isopropyl ether, and DMI means 1,3-dimethyl-2-imidazolidinone.

Furthermore, "%" is based on mass unless specified otherwise.

### <Example 1>

### Preparation of N-(2-bromo-4-(perfluoropropan-2-yl)-6-(trifluoromethyl)phenyl)-2-fluoro-3-(N-methylbenzamide)benzamide (Compound No. 7-1574).

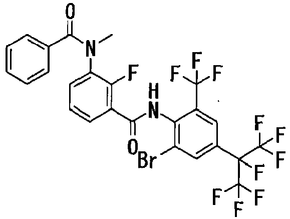

### <1-1>

### Preparation of 4-(perfluoropropan-2-yl)-2-(trifluoromethyl)aniline

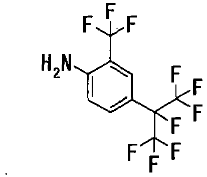

100 g (0.608 mol) of 2-(trifluoromethyl)aniline, 131 g (0.639 mol) of 85% sodium hydrosulfite, and 20.9 g (0.0608 mol) of tetrabutylammonium hydrogen sulfate were charged to a mixed solution of 1500 ml of ethyl acetate and 1500 ml of water, and 53.9 g (0.639 mol) of sodium hydrogen carbonate was added thereto. 198 g (0.669 mol) of heptafluoroisopropyl iodide was added dropwise thereto at room temperature, followed by stirring at room temperature for 6 hours. After the liquid separation, the solvent of the organic layer was evaporated under reduced pressure, and 500 ml of ethyl acetate was charged thereto. 160 g (0.608 mol) of a 4 M hydrogen chloride/ethyl acetate solution was added dropwise thereto, followed by stirring at room temperature for 30 minutes, and then stirring at 5°C for 1 hour. After the filtration, the filtrate was washed with water and a saturated aqueous sodium hydrogen carbonate in this order, and then dried over anhydrous magnesium sulfate, and the solvent was evaporated under reduced pressure. The obtained residue was purified by silica gel column chromatography (eluent solvent; hexane : ethyl acetate=10:1) to prepare 60.0 g (yield 30%) of a target compound.
¹H-NMR (CDCl₃, ppm) δ 4.49 (2H, broad-s), 6.81 (1H, d, J=8.3 Hz), 7.48 (1H, d, J=8.3 Hz), 7.64 (1H, s).

### <1-2>

### Preparation of 2-bromo-4-(perfluoropropan-2-yl)-6-(trifluoromethyl)aniline

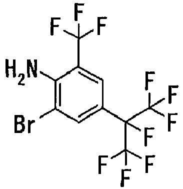

100 g (0.273 mol) of 4-(perfluoropropan-2-yl)-2-(trifluoromethyl)aniline was charged to 500 ml of DMF, and 52.1 g (0.287 mol) of N-bromosuccinimide was charged in separate portions thereto over 30 minutes. After stirring at 60°C for 2 hours, and then cooling to room temperature, the mixture was discharged to 2000 ml of water. The mixture was extracted with ethyl acetate, then washed with saturated brine, and dried over anhydrous magnesium sulfate. The solvent was evaporated under reduced pressure and the obtained residue was purified by silica gel column chromatography (eluent solvent; hexane : ethyl acetate=20:1) to prepare 89.0 g (yield 80%) of a target compound.
¹H-NMR (CDCl₃, ppm) δ 5.03 (2H, broad-s), 7.61 (1H, s), 7.79 (1H, s).

### <1-3>

### Preparation of N-(2-bromo-4-(perfluoropropan-2-yl)-6-(trifluoromethyl)phenyl)-2-chloro-3 -nitrobenzamide

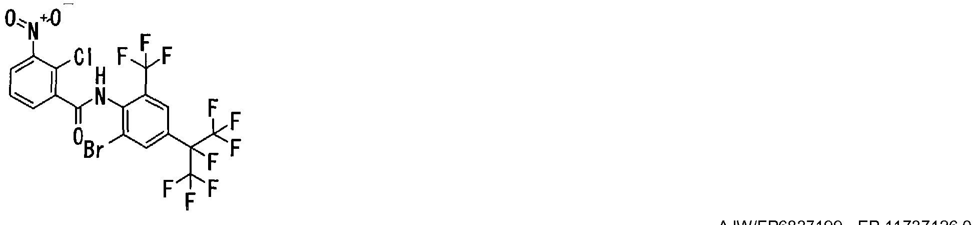

3.60 g (8.82 mmol) of 2-bromo-4-(perfluoropropan-2-yl)-6-(trifluoromethyl)aniline was charged to 20 ml of anhydrous THF, and cooled to -70°C under a nitrogen atmosphere. 4.85 ml (9.70 mmol) of a 2.0 M lithium diisopropyl amide hexane solution was added dropwise thereto, then 2.34 g (10.7 mmol) of acid chloride which was prepared from 2-chloro-3-nitrobenzoic acid and thionyl chloride was dissolved in 5 ml of anhydrous THF, and was added dropwise thereto, followed by stirring at -70°C for 30 minutes and then stirring at room temperature for 30 minutes. The mixture was discharged to an aqueous ammonium chloride solution, then extracted with ethyl acetate, and dried over anhydrous magnesium sulfate. The solvent was evaporated under reduced pressure and the obtained residue was purified by silica gel column chromatography (eluent solvent; hexane : ethyl acetate=10:1→8:2→3:1) to prepare 1.76 g (yield: 34%) of a target compound.
¹H-NMR (CDCl₃, ppm) δ 7.61 (1H, t, J=7.8 Hz), 7.67 (1H, broad-s), 7.93-7.97 (3H, m), 8.18 (1H, broad-s).

### <1-4>

### Preparation of N-(2-bromo-4-(perfluoropropan-2-yl)-6-(trifluoromethyl)phenyl)-2-fluoro-3-nitrobenzamide

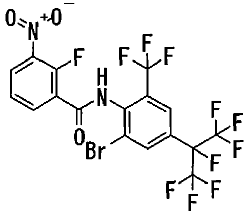

To a solution of N-(2-bromo-4-(perfluoropropan-2-yl)-6-(trifluoromethyl)phenyl)-2-chloro-3-nitrobenzamide 4.89 g (8.27 mmol) in 50 ml of anhydrous DMF was added 2.40 g (41.3 mmol) of potassium fluoride (spray-dried product) under a flow of nitrogen, followed by stirring at 130°C for 10 hours. Liquid separation was carried out by adding ethyl acetate, hexane, and water to the reaction mixture, and then the organic layer was washed with water and saturated brine, and dried over anhydrous magnesium sulfate. The solvent was evaporated under reduced pressure and the obtained residue was purified by silica gel column chromatography (eluent solvent; hexane : ethyl acetate=10:1) to prepare 0.940 g (yield 20%) of a target compound.
¹H-NMR (CDCl₃, ppm) δ 7.53 (1H, t, J=7.3 Hz), 7.93 (1H, broad-s), 8.17-8.18 (2H, m), 8.28-8.32 (1H, m), 8.44-8.48 (1H, m).

### <1-5>

### Preparation of 3-amino-N-(2-bromo-4-(perfluoropropan-2-yl)-6-(trifluoromethyl)phenyl)-2-fluorobenzamide

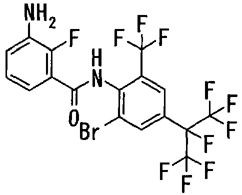

0.940 g (1.63 mmol) of N-(2-bromo-4-(perfluoropropan-2-yl)-6-(trifluoromethyl)phenyl)-2-fluoro-3-nitrobenzamide and 0.960 g (5.05 mmol) of stannous chloride (anhydrous) were added to 10 ml of ethanol, and 1.02 ml (9.78 mmol) of concentrated hydrochloric acid was added thereto, followed by stirring at 60°C for 4 hours. The reaction mixture was adjusted to pH 10 by the addition of an aqueous sodium hydroxide solution, and the precipitated insolubles were removed by filtration using Celite. The filtrate on Celite was washed with ethyl acetate. The filtrate was extracted with ethyl acetate, and the organic layer was washed with a 20% aqueous sodium hydroxide solution and saturated brine, and then dried over anhydrous magnesium sulfate. The solvent was evaporated under reduced pressure and the obtained residue was purified by silica gel column chromatography (eluent solvent; hexane : ethyl acetate=4:1) to prepare 0.930 g (yield 99%) of a target compound.
¹H-NMR (CDCl₃, ppm) δ 3.93 (2H, broad-s), 6.99-7.04 (1H, m), 7.11 (1H, t, J=7.8 Hz), 7.47-7.49 (1H, m), 7.91 (1H, s), 8.14 (1H, s), 8.28 (1H, d, J=14.6 Hz).

### <1-6>

### Preparation of N-(2-bromo-4-(perfluoropropan-2-yl)-6-(trifluoromethyl)phenyl)-2-fluoro-3-(methylamino)benzamide

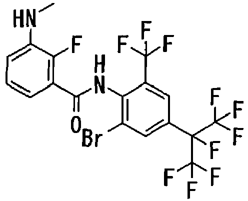

0.930 g (1. 71 mmol) of 3-amino-N-(2-bromo-4-(perfluoropropan-2-yl)-6-(trifluoromethyl)phenyl)-2-fluorobenzamide was added to 5 ml of concentrated sulfuric acid, and 10 ml of a 37% aqueous formaldehyde solution was charged dropwise thereto at 40°C. The reaction mixture was poured into ice-water, adjusted to pH 10 using an aqueous sodium hydroxide solution, and extracted with the addition of ethyl acetate. The organic layer was washed with a 20% aqueous sodium hydroxide solution and saturated brine, and then dried over anhydrous magnesium sulfate. The solvent was evaporated under reduced pressure and the obtained residue was purified by silica gel column chromatography (eluent solvent; hexane : ethyl acetate=8:1) to prepare 0.690 g (yield 72%) of a target compound.
¹H-NMR (CDCl₃, ppm) δ 2.94 (3H, s), 4.14 (1H, broad-s), 6.88-6.93 (1H, m), 7.18 (1H, t, J=7.8 Hz), 7.37-7.41 (1H, m), 7.90 (1H, s), 8.13 (1H, s), 8.27 (1H, d, J=14.6 Hz).

### <1-7> Preparation of N-(2-bromo-4-(perfluoropropan-2-yl)-6-(trifluoromethyl)phenyl)-2-fluoro-3-(N-methylbenzamide)benzamide (Compound No. 7-1574)

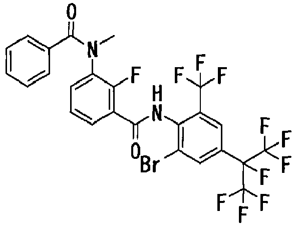

To a solution of 1.54 g (2.75 mmol) of N-(2-bromo-4-(perfluoropropan-2-yl)-6-(trifluoromethyl)phenyl)-2-fluoro-3-(methylamino)benzamide and 0.330 g (4.13 mmol) of pyridine in 5 ml of THF was added 0.460 g (3.30 mmol) of benzoyl chloride, followed by stirring at 60°C for 5 hours. To the reaction mixture were added water and ethyl acetate, and the organic layer was washed with 1 M hydrochloric acid, a saturated aqueous sodium hydrogen carbonate solution, and saturated brine, and then dried over anhydrous magnesium sulfate. The solvent was evaporated under reduced pressure and the obtained residue was purified by silica gel column chromatography (eluent solvent; hexane : ethyl acetate=8:1), and the obtained solid was washed with IPE to prepare 1.45 g (yield 80%) of a target compound.
¹H-NMR (CDCl₃, ppm) δ 3.50 (3H, s), 6.99-7.33 (6H, m), 7.43-7.45 (1H, m), 7.90 (1H, s), 7.97-8.06 (2H, m), 8.13 (1H, s).

### <Example 2>

### Preparation of N-(2,6-dibromo-4-(perfluoropropan-2-yl)phenyl)-2-fluoro-3-(N-methylbenzamide)benzamide (Compound No. 7-1104)

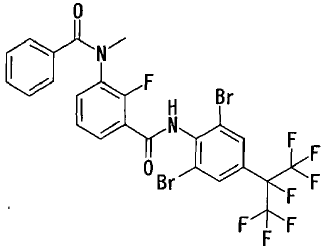

### <2-1>

### Preparation of 4-(perfluoropropan-2-yl)aniline

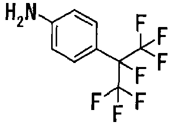

100 g (1.02 mol) of aniline, 230 g (1. 12 mol) of 85% sodium hydrosulfite, and 35.1 g (0.100 mol) of tetrabutylammonium hydrogen sulfate were charged to a mixed solution of 1500 ml of t-butyl methyl ether and 1500 ml of water, and 94.7 g (1.12 mol) of sodium hydrogen carbonate was added thereto. 350 g (1.12 mol) of heptafluoroisopropyl iodide was added dropwise thereto at room temperature, followed by stirring at room temperature for 6 hours. After the liquid separation, the organic layer was washed with 1 M hydrochloric acid, water, and a saturated aqueous sodium hydrogen carbonate solution, and then dried over anhydrous sodium sulfate. The solvent was evaporated under reduced pressure, and 500 ml of ethyl acetate was charged thereto. 255 g (1.02 mol) of a 4 M hydrogen chloride/ethyl acetate solution was added dropwise thereto, followed by stirring at room temperature for 30 minutes and at 5°C for 1 hour. The precipitated solid was separated by filtration, and the solid was charged to 1000 ml of ethyl acetate, adjusted to pH 8 to 9 by the addition of 1000 ml of a saturated aqueous sodium hydrogen carbonate solution at 20°C or lower, and subjected to liquid separation. The organic layer was dried over anhydrous sodium sulfate, and then the solvent was evaporated under reduced pressure to prepare 188 g (yield 71%) of a target compound.
¹H-NMR (CDCl₃, ppm) δ 3.92 (2H, broad-s), 6.69-6.74 (2H, m), 7.35 (2H, d, J=9.3 Hz).

### <2-2>

### Preparation of 2,6-dibromo-4-(perfluoropropan-2-yl)aniline

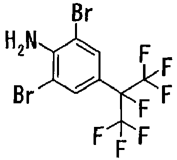

216 g (0.802 mol) of 4-(perfluoropropan-2-yl)aniline was charged to 863 ml of DMF, followed by cooling to 5°C. 285 g (1.60 mol) of N-bromosuccinimide was charged in separate portions thereto over 1 hour. The mixture was stirred at room temperature for 1 hour and then stirred at 37°C for 2 hours. The mixture was discharged to 2000 ml of water, extracted with 2000 ml of ethyl acetate, and washed with 1000 ml of saturated brine. After dried over anhydrous magnesium sulfate, the solvent was evaporated under reduced pressure. The obtained residue was purified by silica gel column chromatography (eluent solvent; hexane : ethyl acetate=20:1) to prepare 304 g (yield 90%) of a target compound.
¹H-NMR (CDCl₃, ppm) δ 4.88 (2H, broad-s), 7.59 (2H, s).

### <2-3>

### Preparation of 2-chloro-N-(2,6-dibromo-4-(perfluoropropan-2-yl)phenyl)-3-nitrobenzamide

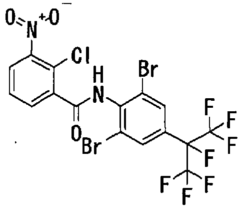

According to the method of 1-3 of Example 1, a target compound was prepared from 2,6-dibromo-4-(perfluoropropan-2-yl)aniline.
¹H-NMR (CDCl₃, ppm) δ 7.58 (1H, t, J=7.8 Hz), 7.66 (1H, broad-s), 7.90 (2H, s), 7.93 (1H, dd, J=1.5, 7.8 Hz), 7.98 (1H, d, J=7.8 Hz).

### <2-4>

### Preparation of N-(2,6-dibromo-4-(perfluoropropan-2-yl)phenyl)-2-fluoro-3-nitrobenzamide

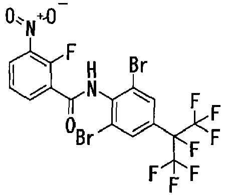

According to the method of 1-4 of Example 1, a target compound was prepared from 2-chloro-N-(2,6-dibromo-4-(perfluoropropan-2-yl)phenyl)-3-nitrobenzamide.
¹H-NMR (CDCl₃, ppm) δ 7.51-7.55 (1H, m), 7.90 (2H, s), 8.16 (1H, d, J=11.7 Hz), 8.27-8.31 (1H, m), 8.48 (1H, t, J=6.3 Hz).

### <2-5>

### Preparation of 3-amino-N-(2,6-dibromo-4-(perfluoropropan-2-yl)phenyl)-2-fluorobenzamide

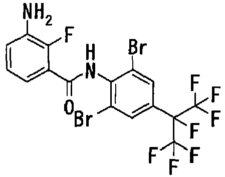

According to the method of 1-5 of Example 1, a target compound was prepared from N-(2,6-dibromo-4-(perfluoropropan-2-yl)phenyl)-2-fluoro-3-nitrobenzamide.
¹H-NMR (CDCl₃, ppm) δ 3. 93 (2H, broad-s), 6. 99-7.04 (1H, m), 7.11 (1H, t, J=7. 8 Hz), 7. 47-7. 49 (1H, m), 7. 91 (1H, s), 8. 14 (1H, s), 8. 28 (1H, d, J=14. 6 Hz).

### <2-6>

### Preparation of N-(2,6-dibromo-4-(perfluoropropan-2-yl)phenyl)-2-fluoro-3-(methylamino)benzamide

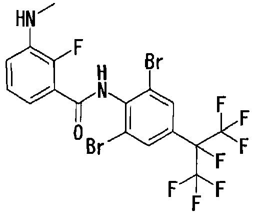

According to the method of 1-6 of Example 1, a target compound was prepared from 3-amino-N-(2,6-dibromo-4-(perfluoropropan-2-yl)phenyl)-2-fluorobenzamide.
¹H-NMR (CDCl₃, ppm) δ 2.94 (3H, d, J=5.6 HZ), 4.87-4.91(1H, m), 6. 91 (1H, t, J=7.9 Hz), 7.18 (1H, t, J=7.9 Hz), 7.41 (1H, t, J=7.1 Hz), 7.87 (2H, s), 8.20 (1H, d, J=13.5 Hz).

### <2-7>

### Preparation of N-(2,6-dibromo-4-(perfluoropropan-2-yl)phenyl)-2-fluoro-3-(N-methylbenzamide)benzamide (Compound No. 7-1104)

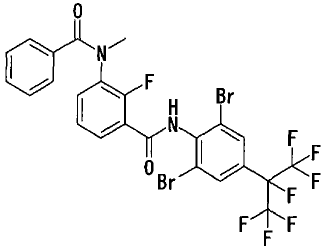

According to the method of 1-7 of Example 1, a target compound was prepared from N-(2,6-dibromo-4-(perfluoropropan-2-yl)phenyl)-2-fluoro-3-(methylamino)benzamide and benzoyl chloride.
¹H-NMR (CDCl₃, ppm) δ 3.51 (3H, s), 7.22-7.44 (7H, m), 7.86 (2H, s), 8.00-8.03 (2H, m).

### <Example3>

### Preparation of 3-benzamide-2-fluoro-N-(4-(perfluoropropan-2-yl)-2,6-bis(trifluoromethyl)phenyl)benzamide (Compound No. 6-2110)

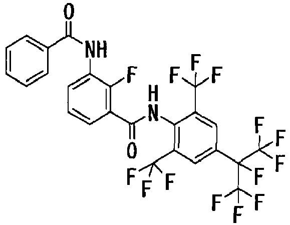

### <3-1>

### Preparation of 2,6-diiodo-4-(perfluoropropan-2-yl)aniline

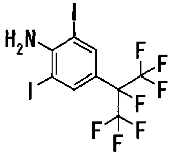

To a solution of 5.74 g (22.0 mmol) of 4-(perfluoropropan-2-yl)aniline obtained in 2-1 of Example 2 in 50 ml of ethanol was added 2.16 g (22.0 mmol) of concentrated sulfuric acid at 5°C. The reaction mixture was warmed to room temperature, and 10.0 g (44.0 mmol) of N-iodosuccinimide was added thereto, followed by stirring for 3 hours. The reaction mixture was neutralized with a saturated aqueous sodium hydrogen carbonate solution. The precipitated crystals were filtered, washed with water, and then dried to prepare 9.00 g (yield 80%) of a target compound.
¹H-NMR (CDCl₃, ppm) δ 4.95 (2H, broad-s), 7.79 (2H, s).

### <3-2>

### Preparation of 2-chloro-N-(2,6-diiodo-4-(perfluoropropan-2-yl)phenyl)-3-nitrobenzamide

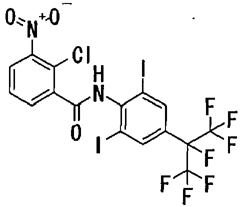

To a solution of 40.0 g (78.0 mmol) of 2,6-diiodo-4-(perfluoropropan-2-yl)aniline in 100 ml of DMI was added 20.6 g (94.0 mmol) of 2-chloro-3-nitrobenzoyl chloride, followed by stirring at 135°C for 3 hours. After cooling to room temperature, the reaction mixture was poured into 1000 ml of water. After extraction with the addition of 1000 ml of ethyl acetate, the organic layer was washed with water and then dried over anhydrous magnesium sulfate. The solvent was evaporated under reduced pressure and the obtained residue was washed with hexane to prepare 56.2 g (yield 99%) of a target compound.
¹H-NMR (CDCl₃, ppm) δ 7.58 (1H, t, J=8.3 Hz), 7.70 (1H, d, J=3.4 Hz), 7.93 (1H, dd, J=1.5, 6.3 Hz), 8.08-8.10 (1H, m), 8.13 (2H, s).

### <3-3>

### Preparation of N-(2,6-diiodo-4-(perfluoropropan-2-yl)phenyl)-2-fluoro-3-nitrobenzamide

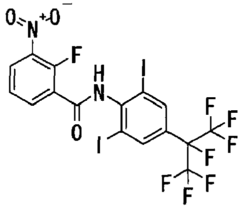

According to the method of 1-4 of Example 1, a target compound was prepared from 2-chloro-N-(2,6-diiodo-4-(perfluoropropan-2-yl)phenyl)-3-nitrobenzamide.
¹H-NMR (CDCl₃, ppm) δ 7.52-7.55 (1H, m), 8.12-8.18 (3H, m), 8.29-8.32 (1H, m), 8.48-8.51 (1H, m).

### <3-4>

### Preparation of 3-amino-N-(2,6-diiodo-4-(perfluoropropan-2-yl)phenyl)-2-fluorobenzamide

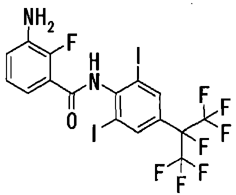

According to the method of 1-5 of Example 1, a target compound was prepared from N-(2,6-diiodo-4-(perfluoropropan-2-yl)phenyl)-2-fluoro-3-nitrobenzamide.
¹H-NMR (CDCl₃, ppm) δ 3.93 (2H, broad-s), 6.99-7.04 (1H, m), 7.08 (1H, t, J=7.8 Hz), 7.39-7.43 (1H, m), 8.10 (2H, s), 8.72 (1H, d, J=11.2 Hz).

### <3-5>

### Preparation of 3-benzamide-N-(2,6-diiodo-4-(perfluoropropan-2-yl)phenyl)-2-fluorobenzamide (Compound No. 6-1260)

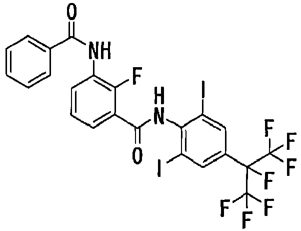

According to the method of 1-7 of Example 1, a target compound was prepared from 3-amino-N-(2,6-diiodo-4-(perfluoropropan-2-yl)phenyl)-2-fluorobenzamide.
¹H-NMR (CDCl₃, ppm) δ 7.39 (1H, t, J=7.8 Hz), 7.52-7.57(4H, m), 7.60-7.63 (2H, m), 7.93-7.94(4H, m), 8.70 (1H, t, J=6.3 Hz).

### <3-6>

### Preparation of 3-benzamide-2-fluoro-N-(4-(perfluoropropan-2-yl)-2,6-bis(trifluoromethyl)phenyl)benzamide (Compound No. 6-2110)

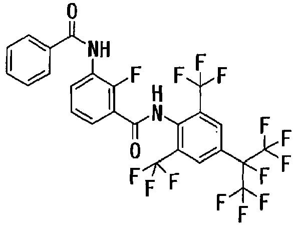

A solution of 1.95 g (2.59 mmol) of 3-benzamide-N-(2,6-diiodo-4-(perfluoropropan-2-yl)phenyl)-2-fluorobenzamide, 0.10 g (0.520 mmol) of copper iodide, and 1.24 g (6.48 mmol) of methyl fluorosulfonyl difluoroacetate in 50 ml of DMF was stirred at 100°C for 6 hours. The reaction mixture was cooled to room temperature, and then 10 ml of water and 100 ml of ethyl acetate were added thereto, followed by filtration through Celite. The organic layer of the liquid was washed with water and a saturated aqueous sodium hydrogen carbonate solution, and then dried over anhydrous magnesium sulfate. The solvent was evaporated under reduced pressure and the obtained residue was purified by silica gel column chromatography (eluent solvent; hexane : ethyl acetate=1:1). The obtained solid was washed with hexane to prepare 0.840 g (yield 51%) of a target compound.
¹H-NMR (CDCl₃, ppm) δ 7.36-7.40 (1H, m), 7.53-7.64 (3H, m), 7.84-7.97 (1H, m), 7.92-7.94 (2H, m), 8.04-8.07 (1H, m), 8.08-8.13 (1H, m), 8.20 (2H, s), 8.68-8.72 (1H, m).

### <Example 4>

### Preparation of 2-fluoro-3-(4-fluoro-N-methylbenzamide)-N-(2-iodo-4-(perfluoropropan-2-yl)-6-(trifluoromethyl)phenyl)benzamide (Compound No. 7-1733)

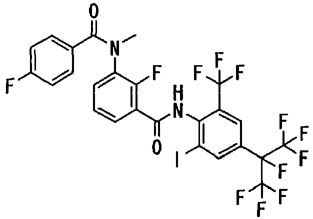

### <4-1>

### Preparation of 2-iodo-4-(perfluoropropan-2-yl)-6-(trifluoromethyl)aniline

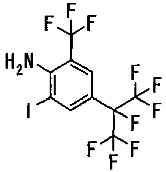

To 1200 mL of ethanol was added 200 g (0.600 mol) of 4-(perfluoropropan-2-yl)-2-(trifluoromethyl)aniline which was obtained in 1-1 of example 1, and 63.2 g (0.630 mol) of concentrated sulfuric acid and 155 g (0.660 mol) of N-iodosuccinimide were added thereto under ice-cooling, followed by stirring at room temperature for 1 hour and 30 minutes and at 40°C for 4 hours. To the reaction solution was added a 4 M aqueous sodium hydroxide solution to neutralize the reaction solution, and then ethyl acetate was added thereto to extract the organic phase. The organic phase was washed with saturated brine, and dried over anhydrous magnesium sulfate, and then the solvent was evaporated under reduced pressure. The obtained residue was purified by silica gel column chromatography (eluent solvent; hexane : ethyl acetate=10:1) to prepare 216 g (yield 80%) of a target compound.
¹H-NMR (CDCl₃, ppm) δ 5.04 (2H, broad-s), 7.64 (1H, s), 7.99 (1H, s).

### <4-2>

### Preparation of 2-chloro-N-(2-iodo-4-(perfluoropropan-2-yl)-6-(trifluoromethyl)phenyl)-3-nitrobenzamide

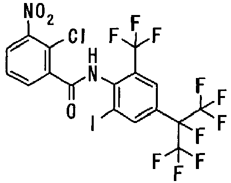

According to the method of 3-2 of Example 3, a target compound was prepared from 2-iodo-4-(perfluoropropan-2-yl)-6-(trifluoromethyl)aniline and 2-chloro-3-nitrobenzoyl chloride.
¹H-NMR (CDCl₃, ppm) δ 7.60 (1H, t, J=7.8 Hz), 7.76 (1H, s), 7.94 (1H, dd, J=1.5, 7.8 Hz), 7.97 (1H, s), 8.03 (1H, dd, J=1.5, 7.8 Hz), 8.39 (1H, s).

### <4-3>

### Preparation of 2-fluoro-N-(2-iodo-4-(perfluoropropan-2-yl)-6-(trifluoromethyl)phenyl)-3-nitrobenzamide

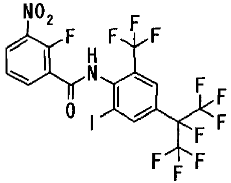

According to the method of 1-4 of Example 1, a target compound was prepared from 2-chloro-N-(2-iodo-4-(perfluoropropan-2-yl)-6-(trifluoromethyl)phenyl)-3-nitrobenzamide.
¹H-NMR (CDCl₃, ppm) δ 7.51-7.55 (1H, m), 7.97 (1H, s), 8.23 (1H, d, J=12.2 Hz), 8.28-8.32 (1H, m), 8.37 (1H, s), 8.44-8.48 (1H, m).

### <4-4>

### Preparation of 3-amino-2-fluoro-N-(2-iodo-4-(perfluoropropan-2-yl)-6-(trifluoromethyl)phenyl)benzamide

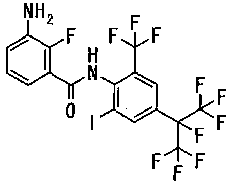

According to the method of 1-5 of Example 1, a target compound was prepared from 2-fluoro-N-(2-iodo-4-(perfluoropropan-2-yl)-6-(trifluoromethyl)phenyl)-3-nitrobenzamide.
¹H-NMR (CDCl₃, ppm) δ 3.92 (2H, broad-s), 7.02-7.04 (1H, m), 7.11 (1H, t, J=7.8 Hz), 7.47-7.52 (1H, m), 7.94 (1H, s), 8.30-8.35 (2H, m).

### <4-5>

### Preparation of 2-fluoro-N-(2-iodo-4-(perfluoropropan-2-yl)-6-(trifluoromethyl)phenyl)-3-(methylamino)benzamide

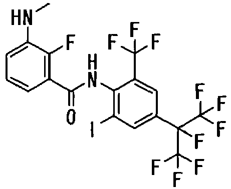

According to the method of 1-6 of Example 1, a target compound was prepared from 3-amino-2-fluoro-N-(2-iodo-4-(perfluoropropan-2-yl)-6-(trifluoromethyl)phenyl)benzamide.
¹H-NMR (CDCl₃, ppm) δ 2.95 (3H, s), 4.15 (1H, broad-s), 6.90 (1H, t, J=8.2 Hz), 7.19 (1H, t, J=7.8 Hz), 7.40 (1H, t, J=7.8 Hz), 7.92 (1H, s), 8.30 (1H, s), 8.34 (1H, s).

### <4-6>

### Preparation of 2-fluoro-3 -(4-fluoro-N-methylbenzamide)-N-(2-iodo-4-(perfluoropropan-2-yl)-6-(trifluoromethyl)phenyl)benzamide (Compound No. 7-1733)

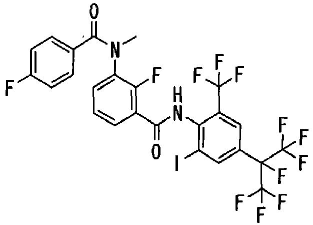

According to the method of 1-7 of Example 1, a target compound was prepared from 2-fluoro-N-(2-iodo-4-(perfluoropropan-2-yl)-6-(trifluoromethyl)phenyl)-3-(methylamino)benzamide and 4-fluorobenzoyl chloride.
¹H-NMR (CDCl₃, ppm) δ 3.50 (3H, s), 6.91 (2H, s), 6.93-7.35 (3H, m), 7.46 (1H, t, J=7.0 Hz), 7.93 (1H, s), 8.01-8.10 (1H, m), 8.13 (1H, broad-s), 8.34 (1H, s).

### <Example 5>

### Preparation of 2-fluoro-3-(3-fluoro-N-methylbenzamide)-N-(2-iodo-4-(perfluoropropan-2-yl)-6-(trifluoromethyl)phenyl)benzamide (Compound No. 7-1732)

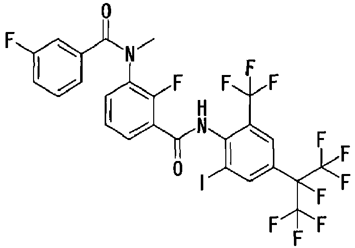

According to the method of 1-7 of Example 1, a target compound was prepared from 2-fluoro-N-(2-iodo-4-(perfluoropropan-2-yl)-6-(trifluoromethyl)phenyl)-3-(methylamino)benzamide which was obtained in 4-5 of example 4 and 3-fluorobenzoyl chloride.
¹H-NMR (CDCl₃, ppm) δ 3.50 (3H, s), 6.91 (2H, s), 7.00-7.18 (4H, m), 7.27-7.31 (1H, m), 7.45-7.48 (1H, m), 7.93 (1H, s), 8.01-8.03 (1H, m), 8.12 (1H, broad-s), 8.34 (1H, s).

### <Example 6>

### Preparation of 2-fluoro-3-(4-fluorobenzamide)-N-(2-iodo-4-(perfluoropropan-2-yl)-6-(trifluoromethyl)phenyl)benzamide (Compound No. 6-1733)

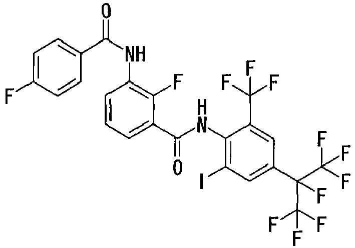

According to the method of 1-7 of Example 1, a target compound was prepared from 3-amino-2-fluoro-N-(2-iodo-4-(perfluoropropan-2-yl)-6-(trifluoromethyl)phenyl)benzamide which was obtained in 4-4 of example 4 and 4-fluorobenzoyl chloride.
¹H-NMR (CDCl₃, ppm) δ 7.19-7.25(2H, m), 7.35 (1H, t, J=7.8 Hz), 7.87-7.97 (4H, m), 8.08 (1H, s), 8.25 (1H, d, J=12.0 Hz), 8.37 (1H, s), 8.65 (1H, t, J=8.0 Hz).

Now, the representative examples of the formulations according to the invention will be shown, although the invention is not limited thereto. "Parts" in Formulation Examples indicates "parts by weight".

### Formulation Example 1 (Emulsion)

10 parts of Compound No. 7-1574 or 7-1733, 6 parts of Sol Pol 355S (surfactant available from Toho Chemical Industry Co.), and 84 parts of Solvesso 150 (available from ExxonMobil Chemical Co.) were homogeneously mixed with stirring to obtain as emulsion the composition for exterminating animal parasites.

### Formulation Example 2 (Ointment)

1 part of Compound No. 7-1574 or 7-1733, 50 parts of white beeswax, and 49 parts of white petrolatum were thoroughly mixed to obtain as ointment the composition for exterminating animal parasites.

### Formulation Example 3 (Tablets)

2 parts of Compound No. 7-1574 or 7-1733, 10 parts of vegetable oil (olive oil), 3 parts of crystalline cellulose, 20 parts of white carbon, and 65 parts of kaolin were thoroughly mixed and then compressed to obtain as tablets the composition for exterminating animal parasites.

### Formulation Example 4 (Injectable)

10 parts of Compound No. 7-1574 or 7-1733, 10 parts of propylene glycol for food additives, 80 parts of vegetable oil (corn oil) were mixed to obtain as injectable the composition for exterminating animal parasites.

### Formulation Example 5 (Solution)

5 parts of Compound No. 7-1574 or 7-1733, 20 parts of surfactant, and 75 parts of ion-exchanged water were thoroughly mixed to obtain as solution the composition for exterminating animal parasites.

Next, the utility of the composition of the invention as a parasiticide will be concretely explained in the following Test Examples, although the invention is not limited thereto.

### <Test Example 1>

### Insecticidal Test for Ctenocephalides Felis

0.5 ml of a solution of acetone, the active ingredient, prepared to the prescribed concentration was added dropwise to a glass tube with a flat bottom (internal diameter: 2.6 cm, bottom area: 5.3 cm², height: 12 cm), and the solvent was evaporated at room temperature to form a dry film of the agent on the bottom surface. About 10 adults of *Ctenocephalides Felis* per glass tube were added, and the adults were contact-exposed to the test compound. After 24 hours of the exposure, the conditions of the adults were examined and classified into "alive" and "dead (including a moribund condition)" to determine the death rate (in triplicate). The results are shown in Table 4.

As the comparative compounds, the following Compounds (A), (B), and (C) disclosed in WO2009/080203, and fipronil were used.

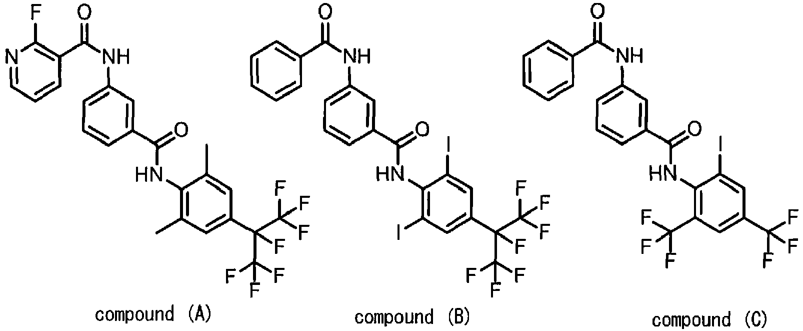

**Table 4**

| active ingredient (compound number) | concentration (µg/ml) | death rate (%) |
|---|---|---|
| 7-1574 | 0.1 | 100.0 |
| 7-1733 | 0.1 | 100.0 |
| 7-1732 | 0.1 | 76. 7 |
| 6-1733 | 0.1 | 70.0 |
| 7-1104 | 0.1 | 73.3 |
| compound (A) | 1.0 | 0.0 |
| compound (B) | 1.0 | 3.2 |
| compound (C) | 1.0 | 0.0 |
| fipronil | 0.1 | 92.1 |
| only acetone | - | 4.7 |

The amide derivatives of Compound No. 7-1574 and 7-1104 were found to be effective even in the examination after 12 hours of the exposure. In addition, they showed immediate effect as compared with the fipronil.

### <Test Example 2>

Insecticidal Test for *Haemaphysalis longicornis* 1 ml of a solution of acetone, the active ingredient, prepared to the prescribed concentration was added dropwise to filter paper inside a petri dish with a diameter of 9 cm, and the acetone was evaporated at room temperature. About 100 larvae of *Haemaphysalis longicornis* per dish were added to the filter paper. The dish was covered by a polyethylene sheet, sealed with a rubber band, and placed in a dark place at a temperature of 25°C and a relative humidity of 100% to expose the larvae to the test substance. After 24 hours of the exposure, the conditions of the larvae were examined and classified into "alive" and "dead (including a moribund condition)".

As a result, the amide derivatives according to the invention were found to be effective in killing *Haemaphysalis longicornis.*

### <Test Example 3>

### Insecticidal Test for Ctenocephalides felis

0.96 ml of a solution of acetone, the active ingredient, prepared to the prescribed concentration was added dropwise to a glass tube with a flat bottom (internal diameter: 3.6 cm, height: 12 cm), and the solvent was evaporated at room temperature to form a dry film of the agent on the bottom surface. About 10 adults of *Ctenocephalides felis* per glass tube were added, and they were contact-exposed to the test compound. After 24 hours of the exposure, the conditions of the adults were examined and classified into "alive" and "dead (including a moribund condition)" to determine the death rate was determined (in triplicate). The results are shown in Table 5.

As the comparative compounds, the following Compounds (D) and (E) disclosed in WO2009/080203, and fipronil were used.

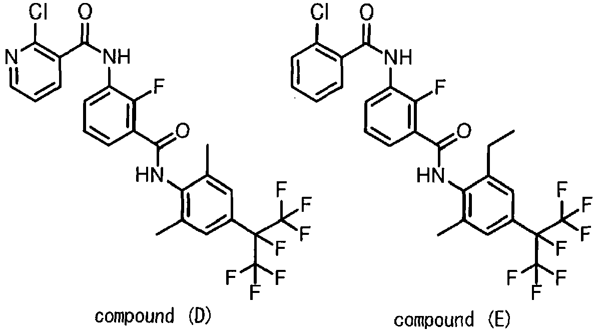

**Table 5**

| active ingredient (compound number) | concentration (µg/ml) | death rate (%) |
|---|---|---|
| 7-1574 | 0.03 | 72.0 |
| | 0.1 | 98. 9 |
| 7-1733 | 0.03 | 21.1 |
| | 0. 1 | 91.6 |
| componud (D) | 0.3 | 22.0 |
| | 1 | 61.4 |
| compound (E) | 1 | 0.0 |
| fipronil | 0.03 | 70.0 |
| | 0. 1 | 100.0 |
| only acetone | - | 1.4 |

### <Test Example 4>

### Insecticidal Test for Rhipicephalus sanguineus

0.5 ml of a solution of acetone, the active ingredient, prepared to the prescribed dose was added dropwise into a glass petri dish with a diameter of 10 cm, and the acetone was evaporated at room temperature. About 10 adults of *Rhipicephalus sanguineus* per dish were added, and the dish was covered by another glass petri dish that was treated with the active ingredient in the same manner described above. After 18 hour exposure to the test substance, the adults were transferred into a glass petri dish that was not treated with the active ingredient. After 48 hours of the exposure, the conditions of the adults were examined and classified into "alive" and "dead (including a moribund condition)" to determine the death rate (in single). The results are shown in Table 6.

As the comparative compounds, said Compounds (B), (C), and (D), and the following Compound (F) disclosed in WO2009/080203, and fipronil were used.

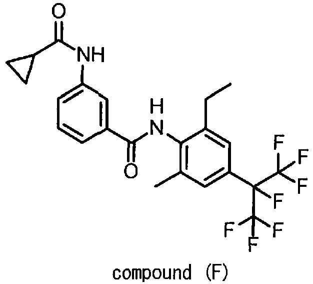

**Table 6**

| active ingredient (compound number) | concentration (mg/m²) | death rate (%) |
|---|---|---|
| 7-1574 | 0.0431 | 10.0 |
| | 0.0861 | 100.0 |
| | 0.172 | 100.0 |
| 7-1733 | 0.0431 | 0.0 |
| | 0.0861 | 80.0 |
| | 0.172 | 100.0 |
| compound (B) | 0.673 | 0.0 |
| | 2.69 | 20.0 |
| | 10.8 | 50.0 |
| compound (C) | 0.673 | 0.0 |
| | 2.69 | 11.1 |
| | 10.8 | 10.0 |
| compound (D) | 0.673 | 0.0 |
| | 2.69 | 10.0 |
| | 10.8 | 50.0 |
| compound (F) | 0.673 | 0.0 |
| | 2.69 | 0.0 |
| | 10.8 | 0.0 |
| fipronil | 0.0431 | 0.0 |
| | 0.172 | 45.5 |
| | 0.673 | 100.0 |

### Industrial Applicability

As the composition for exterminating animal parasites according to the invention has excellent activity for exterminating animal parasites, the composition has wide industrial applicability.

## Claims

1. A composition for use in a method of treatment, wherein the method is for exterminating animal parasites, the composition comprising, as an active ingredient, at least one amide derivative represented by the following Formula (1): 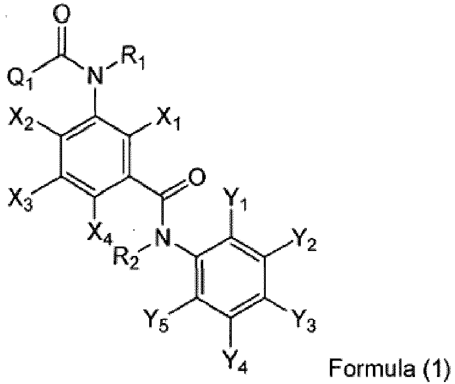 wherein, in Formula (1), Q₁ represents a phenyl group or a phenyl group substituted with a halogen atom; X₁ represents a fluorine atom; X₂, X₃, and X₄ each represent a hydrogen atom; R₁ represents a hydrogen atom or a C1-C3 alkyl group; R₂ represents a hydrogen atom; Y₁ and Y₅ each independently represent a halogen atom or a C1-C3 haloalkyl group; Y₂ and Y₄ each represent a hydrogen atom; and Y₃ represents a heptafluoroisopropyl.

2. The composition for use according to claim 1, wherein in Formula (1), Q₁ represents a phenyl group or a phenyl group substituted with a single fluorine atom; R₁ represents a hydrogen atom or a methyl group; and Y₁ and Y₅ each independently represent a bromine atom, an iodine atom or a trifluoromethyl group.

3. The composition for use according to claim 2, wherein the amide derivative represented by Formula (1) comprises 2-fluoro-3-(N-methylbenzamide)-N-(2-bromo-6-trifluoromethyl-4-(heptafluoropropan-2-yl)phenyl)benzamide, 2-fluoro-3-(4-fluoro-N-methylbenzamide)-N-(2-iodo-6-trifluoromethyl-4-(heptafluoropropan-2-yl)phenyl)benzamide, 2-fluoro-3-(3-fluoro-N-methylbenzamide)-N-(2-iodo-6-trifluoromethyl-4-(heptafluoropropan-2-yl)phenyl)benzamide, 2-fluoro-3-(4-fluorobenzamide)-N-(2-iodo-6-trifluoromethyl-4-(heptafluoropropan-2-yl)phenyl)benzamide, or 2-fluoro-3-(N-methylbenzamide)-N-(2,6-dibromo-4-(heptafluoropropan-2-yl)phenyl)benzamide.

4. The composition for use according to any one of claims 1 to 3, wherein the animal parasite is an ectoparasite.

5. The composition for use according to claim 4, wherein the ectoparasite is a Siphonaptera pest.

6. The composition for use according to claim 4, wherein the ectoparasite is an Acarina pest.

7. A method for exterminating animal parasites, comprising exposing the animal parasite to the composition as defined in any one of claims 1 to 3, wherein the method is not a method of treatment of the human or animal body.

8. The method for exterminating animal parasites according to claim 7, wherein the animal parasite is an ectoparasite.

9. The method for exterminating animal parasites according to claim 8, wherein the ectoparasite is a Siphonaptera pest.

10. The method for exterminating animal parasites according to claim 8, wherein the ectoparasite is an Acarina pest.

## Patentansprüche

1. Zusammensetzung zur Verwendung in einem Behandlungsverfahren, wobei das Verfahren zur Vernichtung von tierischen Parasiten dient, wobei die Zusammensetzung als Wirkstoff zumindest ein durch die folgende Formel (1) dargestelltes Amidderivat umfasst: 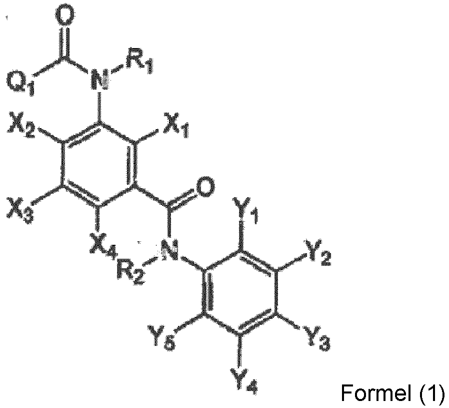 wobei in Formel (1) Q₁ für eine Phenylgruppe oder eine mit einem Halogenatom substituierte Phenylgruppe steht; X₁ für ein Fluoratom steht; X₂, X₃ und X₄ jeweils für ein Wasserstoffatom stehen; R₁ für ein Wasserstoffatom oder eine C1-C3-Alkylgruppe steht; R₂ für ein Wasserstoffatom steht; Y₁ und Y₅ jeweils unabhängig voneinander für ein Halogenatom oder eine C1-C3-Halogenalkylgruppe stehen; Y₂ und Y₄ jeweils für ein Wasserstoffatom stehen; und Y₃ für ein Heptafluorisopropyl steht.

2. Zusammensetzung zur Verwendung nach Anspruch 1, wobei in Formel (1) Q₁ für eine Phenylgruppe oder eine mit einem einzelnen Fluoratom substituierte Phenylgruppe steht; R₁ für ein Wasserstoffatom oder eine Methylgruppe steht; und Y₁ und Y₅ jeweils unabhängig voneinander für ein Bromatom, ein lodatom oder eine Trifluormethylgruppe stehen.

3. Zusammensetzung zur Verwendung nach Anspruch 2, wobei das durch die Formel (1) dargestellte Amidderivat 2-Fluor-3-(N-methylbenzamid)-N-(2-brom-6-trifluormethyl-4-(heptafluorpropan-2-yl)phenyl)benzamid, 2-Fluor-3-(4-fluor-N-methylbenzamid)-N-(2-iod-6-trifluormethyl-4-(heptafluorpropan-2-yl)phenyl)benzamid, 2-Fluor-3-(3-fluor-N-methylbenzamid)-N-(2-iod-6-trifluormethyl-4-(heptafluorpropan-2-yl)phenyl)benzamid, 2-Fluor-3-(4-fluorbenzamid)-N-(2-iod-6-trifluormethyl-4-(heptafluorpropan-2-yl)phenyl)benzamid oder 2-Fluor-3-(N-methylbenzamid)-N-(2,6-dibrom-4-(heptafluorpropan-2-yl)phenyl)benzamid ist.

4. Zusammensetzung zur Verwendung nach einem der Ansprüche 1 bis 3, wobei der tierische Parasit ein Ektoparasit ist.

5. Zusammensetzung zur Verwendung nach Anspruch 4, wobei der Ektoparasit ein Siphonaptera-Schädling ist.

6. Zusammensetzung zur Verwendung nach Anspruch 4, wobei der Ektoparasit ein Acarina-Schädling ist.

7. Verfahren zur Vernichtung von Tierparasiten, welches das Aussetzen des tierischen Parasiten gegenüber einer Zusammensetzung wie in einem der Ansprüche 1 bis 3 definiert umfasst, wobei das Verfahren kein Verfahren zur Behandlung eines menschlichen oder tierischen Körpers ist.

8. Verfahren zur Vernichtung von Tierparasiten nach Anspruch 7, wobei der tierische Parasit ein Ektoparasit ist.

9. Verfahren zur Vernichtung von Tierparasiten nach Anspruch 8, wobei der Ektoparasit ein Siphonaptera-Schädling ist.

10. Verfahren zur Vernichtung von Tierparasiten nach Anspruch 8, wobei der Ektoparasit ein Acarina-Schädling ist.

## Revendications

1. Composition à utiliser dans un procédé de traitement,
dans lequel le procédé consiste à exterminer des parasites d'animal, la composition comprenant, comme ingrédient actif, au moins un dérivé d'amide représenté par la formule (1) suivante : 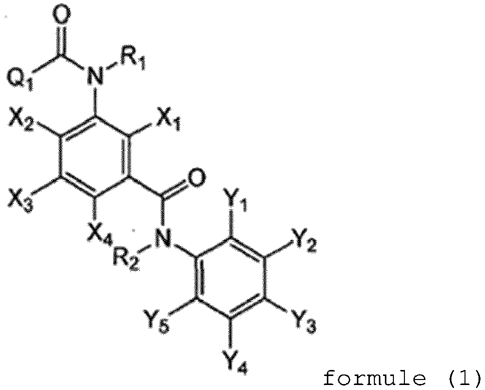 dans laquelle, dans la formule (1), Q₁ représente un groupe phényle ou un groupe phényle substitué par un atome d'halogène ; X₁ représente un atome de fluor ; X₂, X₃ et X₄ représentent chacun un atome d'hydrogène ; R₁ représente un atome d'hydrogène ou un groupe alkyle en C1-C3 ; R₂ représente un atome d'hydrogène ; Y₁ et Y₅ représentent chacun un atome d'halogène ou un groupe haloalkyle en C1-C3 ; Y₂ et Y₄ représentent chacun un atome d'hydrogène ; et Y₃ représente un heptafluoroisopropyle.

2. Composition à utiliser selon la revendication 1, dans laquelle dans la formule (1), Q₁ représente un groupe phényle ou un groupe phényle substitué par un seul atome de fluor ; R₁ représente un atome d'hydrogène ou un groupe méthyle ; et Y₁ et Y₅ représentent indépendamment un atome de brome, un atome d'iode ou un groupe trifluorométhyle.

3. Composition à utiliser selon la revendication 2, dans laquelle le dérivé d'amide représenté par la formule (1) comprend du 2-fluoro-3-(N-méthylbenzamide)-N-(2-bromo-6-trifluorométhyl-4-(heptafluoropropan-2-yl)phényl)benzamide, du 2-fluoro-3-(4-fluoro-N-méthylbenzamide)-N-(2-iodo-6-trifluorométhyl-4-(heptafluoropropan-2-yl)phényl)benzamide, du 2-fluoro-3-(3-fluoro-N-méthylbenzamide)-N-(2-iodo-6-trifluorométhyl-4-(heptafluoropropan-2-yl)phényl)benzamide, du 2-fluoro-3-(4-fluorobenzamide)-N-(2-iodo-6-trifluorométhyl-4-(heptafluoropropan-2-yl)phényl)benzamide ou du 2-fluoro-3-(N-méthylbenzamide)-N-(2,6-dibromo-4-(heptafluoropropan-2-yl)phényl) benzamide.

4. Composition à utiliser selon l'une quelconque des revendications 1 à 3, dans laquelle le parasite d'animal est un ectoparasite.

5. Composition à utiliser selon la revendication 4, dans laquelle l'ectoparasite est un nuisible Siphonaptera.

6. Composition à utiliser selon la revendication 4, dans laquelle l'ectoparasite est un nuisible Acarina.

7. Procédé pour exterminer des parasites d'animal, comprenant l'exposition du parasite d'animal à la composition telle que définie dans l'une quelconque des revendications 1 à 3, dans lequel le procédé n'est pas un procédé de traitement du corps humain ou animal.

8. Procédé pour exterminer des parasites d'animal selon la revendication 7, dans lequel le parasite d'animal est un ectoparasite.

9. Procédé pour exterminer des parasites d'animal selon la revendication 8, dans lequel l'ectoparasite est un nuisible Siphonaptera.

10. Procédé pour exterminer des parasites d'animal selon la revendication 8, dans lequel l'ectoparasite est un nuisible Acarina.
